# FASCICULE DE BREVET EUROPEEN

(11) **EP 1 056 433 B1**
(45) Date de publication et mention de la délivrance du brevet: **11.05.2005**
(21) Numéro de dépôt: 99961139.5
(22) Date de dépôt: 22.12.1999
(51) Int. Cl.: A61K 7/13

(54) **PROCEDE DE TEINTURE METTANT EN OEUVRE UN COMPOSE A METHYLENE ACTIF SPECIFIQUE ET UN COMPOSE CHOISI PARMI UN ALDEHYDE SPECIFIQUE, UNE CETONE SPECIFIQUE, UNE QUINONE ET UN DERIVE DE LA DI-IMINO-ISOINDOLINE OU DE LA 3-AMINO-ISOINDOLONE**
FÄRBUNGSVERFAHREN UNTER VERWENDUNG VON EINER SPEZIFISCHEN AKTIVEN METHYLENVERBINDUNG UND EINER VERBINDUNG AUGEWÄHLT AUS EINEM SPEZIFISCHEN ALDEHYD, EINEM SPEZIFISCHEN KETON, EINEM CHINON UND EINEM DIIMINO-ISOINDOLIN- ODER 3-AMINOISOINDOLONDERIVAT
DYEING METHOD USING A SPECIFIC ACTIVE METHYLENE COMPOUND AND A COMPOUND SELECTED AMONG A SPECIFIC ALDEHYDE, A SPECIFIC KETONE, A QUINONE AND A DI-IMINO-ISOINDOLINE OR 3-AMINO-ISOINDOLONE DERIVATIVE

(30) Priorité: 23.12.1998 FR 9816379
(43) Date de publication de la demande: 06.12.2000
(73) Titulaire: L'OREAL, 75008 Paris (FR)
(72) Inventeur: ANDREAN, Hervé, F-75014 Paris (FR); LAGRANGE, Alain, F-77700 Coupvray (FR)
(74) Mandataire: Casalonga, Axel
(86) Numéro de dépôt international: PCT/FR1999/003245
(87) Numéro de publication internationale: WO 2000/038638

(56) Documents cités:
- EP-A- 0 847 749
- EP-A- 0 873 745
- DE-A- 4 314 317

## Description

La présente invention est relative à l'utilisation pour la teinture des fibres kératiniques d'au moins un composé à méthylène actif spécifique et d'au moins un composé choisi parmi un aldéhyde, une cétone, une quinone et un dérivé de la di-imino-isoindoline ou de la 3-amino-isoindolone, aux compositions tinctoriales comprenant l'association de ces composés, aux procédés de teinture mettant en oeuvre lesdits composés et à un dispositif à plusieurs compartiments renfermant ces composés.

Pour la teinture des fibres kératiniques, et en particulier des fibres kératiniques humaines telles que les cheveux, il est connu d'utiliser des colorants directs ou substances colorées confèrant aux fibres une coloration temporaire ou semi-permanente, de faible puissance tinctoriale et qui s'élimine généralement aux lavages ou à la transpiration. Les gammes des nuances obtenues par ces procédés directs sont en général réduites. Il est également connu d'utiliser des colorants d'oxydation (bases d'oxydation et coupleurs) qui sont des composés initialement incolores ou faiblement colorés, engendrant sous l'action d'un oxydant, des composés colorés par un processus de condensation oxydative. Les colorations d'oxydation sont, comparativement aux colorations directes, permanentes, puissantes, et résistantes aux agents extérieurs (lumière, intempéries, lavages, transpiration et frottements). Néanmoins, l'utilisation de l'agent oxydant peut altérer les fibres kératiniques et rend les procédés de mise en oeuvre des teintures oxydatives relativement complexes.

La demanderesse vient de découvrir un nouveau procédé de teinture, ne mettant pas oeuvre un processus de développement de colorants par voie oxydative, permettant d'obtenir une large gamme de nuances.

Les composés utilisés par la demanderesse sont de petites molécules qui peuvent facilement pénétrer dans la kératine. La demanderesse a constaté, de façon surprenante, que ces composés peuvent ensuite se condenser en chromophores ou colorants, molécules plus volumineuses qui restent piégées au sein de la kératine.

La demanderesse a ainsi constaté que les colorations obtenues sont résistantes aux shampooings et à la transpiration, stables à la lumière, aux intempéries et aux agents chimiques. En quelque sorte, la demanderesse a découvert un nouveau procédé de teinture présentant les avantages de la teinture dite d'oxydation sans en présenter les inconvénients, aucun agent oxydant n'étant utilisé.

La présente invention a donc pour objet l'utilisation pour la teinture des fibres kératiniques d'un composé à méthylène actif spécifique et d'un composé choisi parmi un aldéhyde spécifique, une cétone spécifique, une quinone et un dérivé de la diimino-isoindoline ou de la 3-amino-isoindolone. La demande EP-A-0 847 749 concerne l'utlisation de dérivés de la di-imino-isoindoline ou de 1,3 amino-isoindolone pour la teinture des fibres kératiniques. Ces composés sont utilisés comme précurseurs de coloration, ils peuvent teindre les fibres keratiniques sans agent oxydant, en présence de composés à fonction aminé primaire ou secondaire.

Un autre objet de l'invention est relatif aux compositions de teintures comprenant ces composés.

La présente invention a aussi pour objet un procédé de teinture des fibres kératiniques consistant à appliquer sur les fibres un composé à méthylène actif spécifique et un composé choisi parmi un aldéhyde spécifique, une cétone spécifique, une quinone et un dérivé de la di-iminoisoindoline ou de la 3-amino-isoindolone, soit simultanément, sous forme d'un mélange extemporané, soit de façon successive.

Un autre objet de l'invention consiste aussi en un agent de teinture pour la mise en oeuvre du procédé de l'invention.

D'autres objets de l'invention apparaîtront à la lumière de la description.

L'objet principal de la présente invention est donc l'utilisation pour la teinture des fibres kératiniques, en particulier des fibres kératiniques humaines telles que les cheveux humains, d'au moins un composé à méthylène actif spécifique et d'au moins un composé choisi parmi un aldéhyde, une cétone, une quinone, et un dérivé de la diimino-isoindoline ou de la 3-amino-isoindolone permettant d'obtenir, par réaction sans agent oxydant une coloration desdites fibres kératiniques.

Dans le cadre de la présente invention, un composé à méthylène actif se définit comme un groupement méthylène substitué par deux groupements à effet électro ou mésomère attracteur. De tels composés sont notamment décrits dans Advanced Organic Chemistry JERRY MARCH, 4ème édition Wiley Interscience pages 279, 741, 795.

Les composés à méthylène actif de la présente invention sont plus particulièrement choisis parmi :
1) les composés de formule (I) suivante: 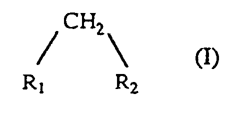 dans laquelle:
   R₁ désigne un groupement -COR ou -COOR avec R désignant un atome d'hydrogène ou un groupement alkyle,
   R₂ désigne les groupements désignés par R₁, un groupement nitrile, un groupement aryle ou alkylaryle substitué ou non, ou un hétérocycle substitué ou non ;
2) les composés de formule (II) suivante: 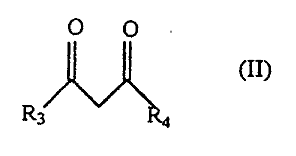 dans laquelle :
   R₃ désigne les groupements désignés par R₂
   R₄ désigne un groupement alkyle substitué ou non, un groupement acétyloxy, un groupement cycloalkyle, un groupement alkylaryle substitué ou non, un groupement aralkyle, un groupement aryle substitué ou non, un groupement aminoaryle substitué ou non, ou un hétérocycle substitué ou non ;
3) les composés de formule (III) suivante: 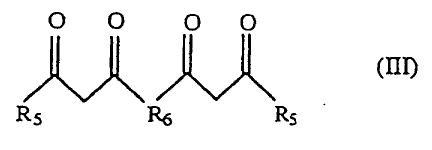 dans laquelle:
   R₅ désigne les groupements désignés par R₂
   R₆ désigne un groupement aryle ou aralkyle substitué ou non, un groupement aminoaryle substitué ou non, ou un hétérocycle substitué ou non ;
4) les dérivés de pyrazole (i) de formules (IV) et (V) suivantes : 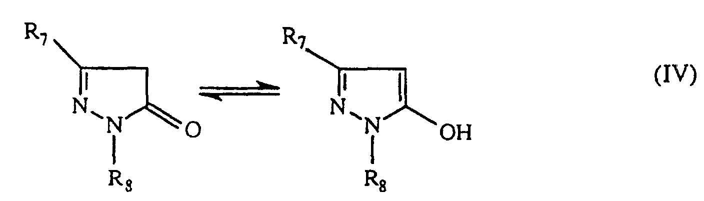 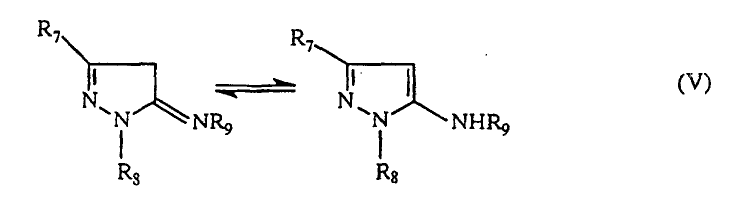 dans lesquelles :
   R₇ et R₈, identiques ou différents, désignent les groupements désignés par R₄,
   R₉ désigne un atome d'hydrogène ou un groupement alkyle substitué ou non;
   et (ii) formés de deux cycles pyrazole de formule (IV) ou (V) reliés par R₇ ou R₈ ;
5) les dérivés d'acides barbituriques (i) de formule (VI) suivante : 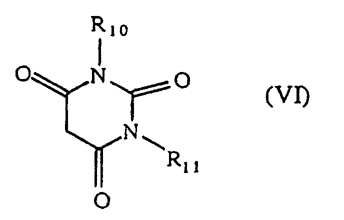 dans laquelle :
   R₁₀ et R₁₁, identiques ou différents, désignent un groupement alkyle substitué ou non, un groupement alcényle, un groupement cycloalkyle, un groupement alkylaryle ou un groupement aryle substitué ou non et (ii) les composés formés de deux cycles de formule (VI) reliés par R₁₀ ou R₁₁ ;
6) Les dérivés de pyridines de formule (VII) : 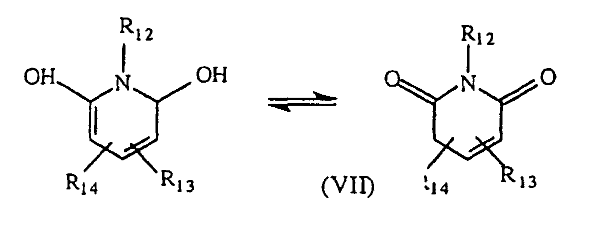 dans laquelle :
   R₁₂ désigne un groupement alkyle substitué ou non, un groupement aryle substitué ou non ;
   R₁₃ désigne un atome d'hydrogène, un groupement alkyle substitué ou non, un groupement aryle substitué ou non
   R₁₄ désigne un atome d'hydrogène, un groupement nitrile, un groupement alkyle substitué ou non, un groupement COOR, R désignant un atome d'hydrogène ou un groupement alkyle substitué ou non.
7) les dérivés de formule (VIII) suivante : 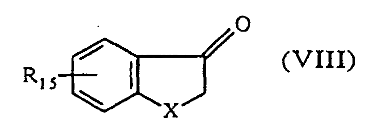 dans laquelle :
   X désigne un atome d'oxygène, de soufre, d'azote ou un groupement
   NR', R' désignant un groupement alkyle,
   R₁₅ désigne un atome d'hydrogène, de chlore, de brome, un groupement hydroxy, nitro, alkyle, alcoxy, carboxamide, sulfonamide ou nitrile.
8) les dérivés de formules (IX) et (X) suivantes: 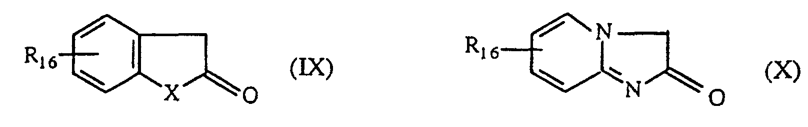 dans lesquelles :
   X désigne un atome d'oxygène, de soufre, d'azote, ou un groupement NR', R' désignant un groupement alkyle,
   R₁₆ désigne les atomes et groupements désignés par R₁₅ ;
9) les dérivés de formule (XI) suivante : 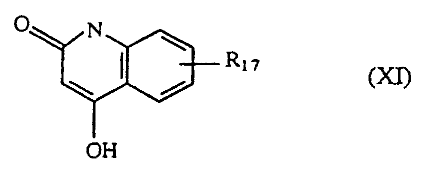 dans laquelle :
   R₁₇ désigne un atome d'hydrogène, un groupement hydroxy ou un groupement alkyle substitué ou non ou un groupement aryle ou alkylaryle substitué ou non.
10) les dérivés d'indandione de formule (XII) suivante: 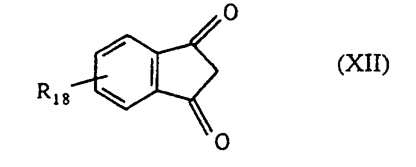 dans laquelle :
   R₁₈ désigne un atome d'hydrogène, de chlore, de brome, un groupement nitro, alkyle, alkoxy, carboxamide, sulfonamide ou nitrile.
11) les dérivés de formule (XIII) suivante : 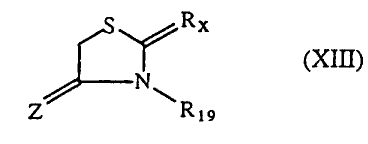 dans laquelle:
   Z désigne O ou NR avec R = H ou alkyle
   Rₓ désigne un atome de soufre, ou NR, R désignant un atome d'hydrogène ou un groupement alkyle ;
   R₁₉ désigne un atome d'hydrogène, un groupement alkyle, alcoxy, nitro ou nitrile ;
12) les dérivés de dioxopyrazole de formule (XIV) suivante: 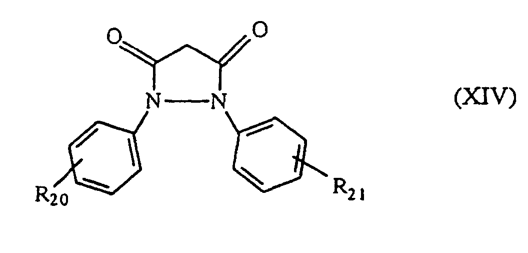 dans laquelle :
   R₂₀ et R₂₁, identiques ou différents, désignent un atome d'hydrogène, un groupement alkyle, alcoxy, nitro ou nitrile.
13) les dérivés de 5-oxoimidazole de formule (XV) suivante : 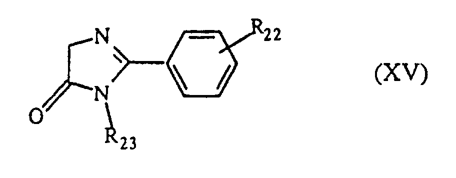 dans laquelle :
   R₂₂ désigne un atome d'hydrogène ou un groupement alkyle
   R₂₃ désigne un atome d'hydrogène, un groupement alkyle, alcoxy, nitro ou nitrile.
14) les dérivés de déhydrobutyrolactone de formule (XVI) suivante: 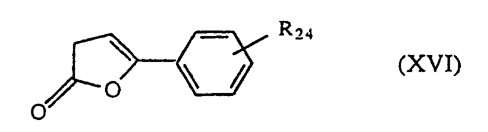 dans laquelle :
   R₂₄ désigne un atome d'hydrogène, un groupement alkyle, alcoxy, nitro ou nitrile.
15) Les composés de formule (XVII) suivante : 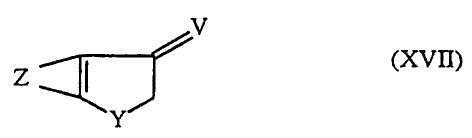 dans laquelle :
   Z forme un cycle aromatique
   V désigne un atome d'oxygène ou un groupement 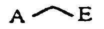
   dans lequel A ou E désigne un substituant ayant une constante de Hammet comprise entre 0,4 et 2,0 ou des substituants dont la somme des constantes de Hammet est comprise entre 0,4 et 2,0
   Y désigne Co, O, S, NR₁ lorsque V est différent d'un atome d'oxygène ou désigne CS, C = NR₂, SO, SO₂, avec R₁ ou R₂ désignant un atome d'hydrogène ou un radical alkyle ;
   et parmi les sels cosmétiquement acceptables des composés définis ci-dessus.

Ils sont utilisés en combinaison avec au moins
un aldéhyde correspondant à la formule (XVIII) suivante: 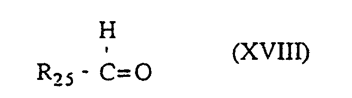 dans laquelle :
R₂₅ désigne un groupement de formule (XVIII A) suivante: 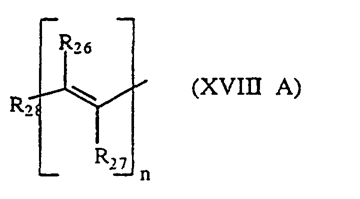
dans laquelle
R₂₆ et R₂₇, identiques ou différents, désignent un atome d'hydrogène, un groupement alkyle, mono ou polyhydroxyalkyle, alkylhydroxyalkyle, alcoxy, -CF₃ ou -OCF₃,
R₂₆ et R₂₇ peuvent également former conjointement avec les atomes auxquels ils sont rattachés un cycle aryle ou un hétérocyclique à 5 ou 6 chaînons, lesdits cycles pouvant être substitués ou non;
n désigne un nombre entier de 0 à 3,
R₂₈ désigne les substituants désignés par R₂₆, un groupement aryle, alkylaryle substitué ou non, un groupe hétérocyclique à 5 ou 6 chaînons substitué ou non,
ou aux sels cosmétiquement acceptables de ces composés;
une cétone correspondant aux formules (XIX) ou (XX) suivantes: 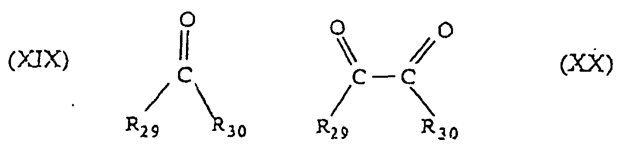 dans lesquelles :
R₂₉ désigne les substituants désignés par R₂₅
R₃₀ désigne un groupement alkyle, mono ou polyhydroxyalkyle, alkyl hydroxyalkyle, un groupement aryle, alkylaryle, un hétérocyclique à 5 ou 6 chaînons substitué ou non,
R₂₉ et R₃₀ peuvent également former conjointement avec les atomes auxquels ils sont rattachés un cycle aryle à 5 ou 6 chaînons, ou un hétérocyclique comprenant des hétéroatomes tels que N ou S, ledit cycle pouvant lui-même être rattaché à un cycle aryle à 5 ou 6 chaînons ou à un hétérocycle comprenant des hétéroatomes tels que N ou S, lesdits cycles pouvant être substitués ou non,
ou aux sels cosmétiquement acceptables de ces composés,
- une quinone et un dérivé de la di-imino-isoindoline, ou de la 3-amino-isoindolone permettant d'obtenir, par réaction sans agent oxydant, une coloration desdites fibres kératiniques.

Parmi les composés de formule (I), (II) et (III) on peut notamment citer l'acide malonique et ses esters, l'acide acetoacétique et ses dérivés.

Les composés de formule (IV) peuvent notamment être les suivants: pyrazolone(5), 3-méthyl-pyrazolone(5), 1-phényl-3-méthyl-pyrazolone(5), 1-(b-cyanéthyl)-3-méthyl-pyrazolone(5), 1,-3diméthyl-pyrazolone(5), 1-(b-acétoxyéthyl)-3-méthyl-pyrazolone(5), 1-(o-chlorophényl)-3-méthyl-pyrazolone(5), 1-phényl-3-carbométhoxy-pyrazolone(5), 1-(3-aminophényl-pyrazolone(5), 1-(4-aminophényl)-pyrazolone(5), 3-méthyl-pyrazolone(5)-1-carboxamide, 1-phényl-pyrazolone(5)-3-carboxamide, amino-pyrazole, 1-phényl-5-amino-pyrazole, 1-benzyl-5-amino-pyrazole, 1-cyclohexyl-5-amino-pyrazole, 1-éthyl-3-méthyl-5-amino-pyrazole, 1-benzyl-3-phényl-5-amino-pyrazole, 1-isopentyl-5-amino-pyrazole, 1-furfuryl-5-amino-pyrazole, 2-méthyl-4H-pyrazolo(5)-[2,3-a]-benzimidazole, [1-(3-thiacyclopentyl)-3-méthyl-pyrazolone(5)-S-dioxyde], 2-méthyl-1H-3,3a, 8-triaza-cyclopenta[a]indene.

Les dérivés d'acides barbituriques de formule (VI) peuvent être choisis parmi les acides di-n-butyl-, di-iso-butyl-, di-N-amyl-, di-iso-amyl-, di-n-hexyl-, di-benzyl-, di-β-phényléthyl-, di-cyclo-hexyl-, di-phényl-, di-p-tolyl-, di-p-méthoxybenzyl-barbittiriques ; les acides N-méthyl-N'-n-butyl-, N-méthyl-N'-benzyl-, N-méthyl-N'-β-phényléthyl-, N-méthyl-N'-γ-phénylpropyl-, N-méthyl-N'-γ-phénylbutyl-, N-méthyl-N'-α-isobutyl-γ-phenylpropyl-, N-méthyl-N'-cyclohéxyl-, N-méthyl-N'-phényl-, N-méthyl-N'-p-tolyl-, N-méthyl-N'-norbornylméthyl-barbituriques et les dérivés N-éthyl et N-n-butyl correspondants.

Les pyridines et pyridones de formule (VII), peuvent, par exemple, être la 2,6-dihydroxy-3-cyano-4-méthyl-pyridine, celles des familles des cyanopyridones, aminonitropyridones et aminocyanopyridones et notamment : la N-méthyl,-3-cyano-4-méthyl-6-hydroxy-pyridone-2, la N-éthyl,-3-cyano-4-méthyl-6-hydroxy-pyridone-2, la N-b-méthoxyéthyl-3-cyano-4-méthyl-6-hydroxy-pyridone- la 2,6-dihydroxy-3-cyano-4-méthyl-pyridine, la N-b-hydroxyéthyl-3-cyano-4-méthyl-6-hydroxy-pyridone-2, la N-butyl,-3-cyano-4-méthyl-6-hydroxy-pyridone-2 et la N-phényl,-3-cyano-4-méthyl-6-hydroxy-pyridone-2.

Les dérivés de formule (VIII) peuvent notamment être choisis parmi le 6-hydroxy-benzofurane-(2H)-one et le benzofurane 2-(H)-one.

Les dérivés de formule (IX) peuvent, par exemple, être :
- 1,3-dihydro-indol-2-one
- 3H-Benzofuran-2-one
- 1-Méthyl-1,3-dihydro-indol-2-one
- 5-Méthoxy-3H-benzofuran-2-one
- 5-Nitro-1,3-dihydro-indol-2-one
- 1-Méthyl-5-nitro-1,3-dihydro-indol-2-one
- 6-Méthoxy-1,3-dihydro-indol-2-one
- 5-Chloro-1,3-dihydro-indol-2-one
- 5,6-Difluoro-1,3-dihydro-indol-2-one
- 6-Hydroxy-5méthoxy-1,3-dihydro-indol-2-one
- 5,6-Diméthoxy-1,3-dihydro-indol-2-one
- 6-Trifluorométhyl-1,3-dihydro-indol-2-one.

Les dérivés de formule (X) peuvent, par exemple, être :
- Imidazo[1,2-a]pyridin-2-one
- 6-Bromo-imidazo[1,2-a)]pyridin-2-one.

Les dérivés de formule (XI) sont préférentiellement choisis parmi les dérivés pour lesquels R₁₇ désigne un atome d'hydrogène comme par exemple la 2,4-dihydroxyquinoléine.

Les dérivés de formule (XII) correspondent notamment à l'1,3-indanedione.

Les dérivés de formule (XIII) sont préférentiellement choisis parmi la rhodamine et la 4-imino-4,5-dihydro-thiazol-2-ylamine.

Comme dérivés de formule (XIV), on peut citer le 1,2-diphényldioxopyrazole.

Les dérivés de formule (XV) sont notamment choisis parmi :
- 2-Phényl-3,5-dihydro-imidazol-4-one
- 3-Méthyl-2-p-tolyl-3,5-dihydro-imidazol-4-one.

Comme composé de formule (XVI), préféré, on peut citer le phényldihydrobutyrolactone.

Comme composés de formule (XVII) préférés on peut citer : 1,1-dioxo-1,2-dihydro-11,6-benzo[b]-thiophen-3-one, 2-(1,1-dioxo-1,2-dihydro-11,6-benzo[b]-thiofen-3-ylidène)-malonitrile.

La quinone peut répondre aux formules (XXI) et (XXII) suivantes: 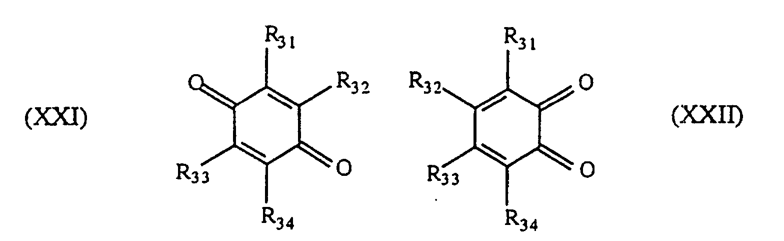 dans lesquelles :
R₃₁ désigne un atome d'hydrogène, d'halogène, un groupement sulfonique ou alcoxy.
R₃₂, R₃₃ et R₃₄, identiques ou différents désignent un atome d'hydrogène, d'halogène, un groupement hydroxy, alkyle, mono ou polyhydroxyalkyle, alkylhydroxyalkyle, alkylsulfonyle, carboxyalkyle, aminoalkyle, alkylaminoalkyle, (di-hydroxy)alkylaminoalkyle, ou alkyle-NR'R" (avec R' et R" désignant alkyle ou pouvant former ensemble avec l'atome d'azote auxquels ils sont rattachés un cycle aryle ou un hétérocycle à 5 ou 6 chaînons), un groupement aryle, un groupe amino pouvant être substitué par un alkyle ou un hydroxyalkyle,
R₃₁ et R₃₂, R₃₁ et R₃₃ ou R₃₃ et R₃₄ peuvent former conjointement avec les atomes auxquels ils sont rattachés un cycle aryle ou un hétérocycle à 5 ou 6 chaînons, substitué ou non;
ou aux sels cosmétiquement acceptables de ces composés.

Les dérivés de la di-imino-isoindoline ou de la 3-amino-isoindolone peuvent être ceux correspondant à la formule (XXIII) suivante: 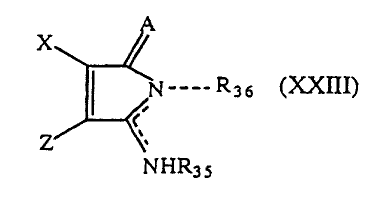 dans laquelle
R₃₅ et R₃₆, identiques ou différents, désignent un atome d'hydrogène, un groupement alkyle, mono ou polyhydroxyalkyle, alkylhydroxyalkyle, aminoalkyle, alkylaminoalkyle, (di-hydroxy)alkylaminoalkyle, ou un groupement alkyle NR'R", avec R' et R" désignant alkyle ou pouvant former conjointement avec l'atome d'azote auxquels ils sont rattachés un cycle aryle ou un hétérocycle à 5 ou 6 chaînons,
A désigne un atome d'oxygène ou NH,
X et Z forment ensemble un cycle aryle ou un hétérocycle à 5 ou 6 chaînons, substitué ou non;
ou aux sels cosmétiquement acceptables de ces composés.

Parmi les composés préférés de formule (XVIII), on peut notamment citer le beuzaldéhyde, les 2,3,4,monohydroxy-benzaldéhydes, les 2,3,4,monométhoxy-benzaldéhydes, les 2,3,4,monométhyl-benzaldéhydes, les (2,3), (2,4), (2,5), (2,6), (3,5)-dihydroxy benzaldéhydes, les (2,3), (2,4), (2,5), (2,6), (3,5)-diméthoxy benzaldéhydes, la vaniline, l'isovaniline, le syringaldéhyde, les ortho, iso, téré-phthaldéhyde, les (2,3), (2,4), (2,5), (2,6), (3,5)-diméthyl-benzaldéhydes, le 4-isopropyl-benzaldéhyde, 4-diméthylamino-benzaldéhyde, 4-diéthylaminol-benzaldéhyde, le pipéronal, les (2,6), (3,5)-diméthyl-4-hydroxy-benzaldéhyde, les 2,3,4-mononitro-benzaldéhydes, le 2-hydroxy-3-méthoxy-benzaldéhyde, le 2-hydroxy-4-méthoxy-benzaldéhyde, le 2-hydroxy-5-méthoxy-benzaldéhyde, le 2-hydroxy-6-méthoxybenzaldéhyde, le 4-méthylthio-benzaldéhyde, les (2,3,4), (2,4,6), (3,4,5), (2,4,5)-trihydroxy-benzaldéhydes, les méthyles 2, 3 et 4-formylbenzoates, les 2,3,4-mono(2-hydroxyethoxy)-benzaldéhydes, le 4-nitro-3-hydroxy-benzaldéhyde, le 3-nitro-4-hydroxy-benzaldéhyde, le 2-nitro-4-hydroxy-benzaldéhyde, le 3-nitro-2-hydroxy-benzaldéhyde, les 2,3,4-monotrifluoro-benzaldéhydes, le 2,3-dihydroxy-4-méthoxy-benzaldéhyde, le 3,4-dihydroxy-5-méthoxy-benzaldéhyde, le 3,5 -dihydroxy-4-méthoxy-benzaldéhyde, le 3-méthoxy-2-nitrobenzaldéhyde, le 4-méthoxy-2-nitrobenzaldéhyde, le 2-méthoxy-3-nitrobenzaldéhyde, le 4-méthoxy-3-nitrobenzaldéhyde, les (2,3,4), (2,4,6), (3,4,5), (2,4,5)-triméthoxy-benzaldéhydes, la 5-nitrovaniline, les (2,4), (2,6)-dinitrobenzaldéhydes, le pentaméthyl-benzaldéhyde, le 4-méthylsulfonyl-benzaldéhyde, les acides 2,3,4-monoformylphénoxyacétiques, le 4-diéthylamino-salicylaldéhyde, le 4(3-diméthylaminopropoxy)-benzaldéhyde, le 2,3-dihydrobenzo (b)furan-5-carboxaldéhyde, le 1 et le 2 naphthaldéhyde, le 6 et 5 carboxaldéhyde-1,4-benzodioxane, les 2,4-monhydroxy-1-naphtaldéhydes, le 1-monhydroxy-2-naphtaldéhyde, le 1(4-formylphényl)-imidazole, le 4-pyrrolidinol-benzaldéhyde, les 2,4 monométhoxy-1-naphthaldéhydes, le 2,3-diméthyl-chroman-6-carboxaldéhyde, le 2,3,6,7-tétrahydro-1H,5H-pyrido(3,2,1-IJ) Quinoline-9-carbaldéhyde, le 4 diméthylamino-1-naphthaldéhyde, le 9-anthraldéhyde, le 3-nitro-4-pyrrolidino-benzaldéhyde, le 3-nitro-4-pipéridino-benzaldéhyde, le 3-nitro-4-morpholino-benzaldéhyde, les pyridines 2,3,4-monocarboxaldéhydes, le 2,6-pyridino-dicarboxaldéhyde, le 5-formyl-6-méthyluracil, le pyridoxale, les quinoléïnes - 2,3,4-monocarboxaldéhydes, le 8-hydroxy-quinoléïne-2-carboxaldéhyde, les 2 et 3-furaldéhydes, les 2 et 3-thiénylcarboxaldéhydes, les 2 et 3-imidazo-carboxaldéhydes, le 2-pyrrolcarboxaldéhyde, le 5-nitro-2-furaldéhyde, le 5-(diméthylamino)-2-furaldéhyde, les 2,5 et 2,3-thiophène-dicarboxaldéhydes, le pyrazol-3-carbaldéhyde, le 5-nitro-2-thiophène-carboxaldéhyde, le 5-nitro-3-thiophènecarboxaldéhyde, l'indole-3-carboxaldéhyde, le N-méthyl-indole-3-carboxaldéhyde, le 2-méthyl-indole-3-carboxaldéhyde, les 4,5,6,7-monométhyl-indole-carboxaldéhyde et l'acide 5-formyl-2-furansulfonique.

Les cétones de formules (XIX) et (XX) peuvent être choisies parmi la 2,3 indolinedione, la 2,3-butanedione, la 2,3-pentanedione, la (2,3), (3,4)-hexanedione, la 1-phényl-1,2-propanedione, le benzyl, le furil, le 2,2'-pyridil, le nitro-benzyl, l'anisil, le 3,3'-diméthoxybenzyl,le 4,4'-bis(diméthylarnino)benzyl, la camphoroquinone, le cyclohexane-1,2-dione, l'isatine, la N-méthyl-isatine, la 4,5,6,7-monométhyl-isatine, la (4,5),(4,7),(5,7),(6,7)-diméthyl-isatine, la N-éthyl-isatine, la N-hydroxyméthyl-isatine, la 5,6,7 monométhoxy-isatine, la 4,5,6,7 monochloro-isatine, la 4,5,6,7 monobromo-isatine, la N-isopropyl-isatine, la N-butyl-isatine, la N-propyl-isatine, la 5-nitro-isatine, l'acide 5-sulfonique-isatine, la 2,4,5-trihydroxypyrimidine, l'alloxane, la 1,3-diméthyl-hexahydro-2,4,5,6-pyrimidinetetraone, la ninhydrine, la chinisatine, le 1,3-indenedione, l'acide squarique, l'acide croconique, la 3,4-diméthoxy-3-cyclobutène-1,2-dione, la 3,4-éthoxy-3-cyclobutène-1,2-dione, la 3,4-isopropoxy-3-cyclobutène-1,2-dione, la 3,4-di-N-butoxy-3-cyclobutène-1,2-dione, l'acide rhodizonique, l'oxindole, la N-méthyl-2-indolinone, la N-méthyl-nitro-2-indolinone, le 6-méthoxyoxindole, le 5,6-diméthoxyoxindole et les 5 et 6-monochlorooxindole.

Les quinones préférées de formules (XXI) et (XXII) sont, entre autres, la 1,4 naphtoquinone, la spinulosine, l'atromentine, l'aurentioglyocladine, la 2,5-dihydroxy-6-méthylbenzoquinone, la 2-hydroxy-3-méthyl-6-méthoxylbenzoquinone, la 2,5-dihydroxy-3,6-diphénylbenzoquinone, la 2,3-diméthyl-5-hydroxy 6-méthoxy-benzoquinone, la 2,5-dihydroxy 6-isopropyl-benzoquinone, la lawsone, la juglone, la fafioline, la naphtazarine, la naphtopurpurine, le lapachol, la plumbagine, la chloroplumbagine, la drosérone, la shikonine, la 2-hydroxy-3-méthyl-1,4-naphtoquinone, la 3,5-dihydroxy-1,4-naphtoquinone, la 2,-5-dihydroxy-1,4-naphtoquinone, la 2-méthaxy-5-hydroxy-1,4-naphtoquinone, la 3-méthoxy-5-hydroxy-1,4-naphtoquinone, la (1,4),(1,2)naphtoquinone, la 4,5-diméthoxy-1,2-benzoquinone, la phenanthrènequinone et l'acide 4-sulfonique(1,2)naphtoquinone.

Les dérivés de formule (XXIII) sont notamment représentés par la 3-imino-3H-isoindol-ylamine, la 3-imino-4-méthyl-3H-isoindol-ylamine, la 3-imino-4-terbutyl-3H-isoindol-1-ylamine, la 3-imino-7-nitro-3H-isoindol-1-ylamine, la 3-amino-1-imino-1H-isoindol-4-ol, la 3-imino-7-isopropoxy-3H-isoindol-1-ylamine, la 3-imino-7-(2,2,2-trifluoroéthoxy)-3H-isoindol-1-ylamine, la 3-imino-7-éthoxy-3H-isoindol-1-ylamine, la 3-imino-7-butoxy-3H-isoindol-1-ylamine, l'acide 3-amino-1-imino-1H-isoindole-4-sulfonique, la 3-imino-7-chloro-3H-isoindol-1-ylamine, la 3-imino-5-méthyl-3H-isoindol-1-yiamine, la 3-imino-5-éthyl-3H-isoindol-1-ylamine, la 3-imino-5-terbutyl-3H-isoindol-1-ylamine, la 3-imino-5-amino-3H-isoindol-1-ylamine, la N-(1-amino-3-imino-3H-isoindol-5-yl)-acétamide, la 3-imino-5-nitro-3H-isoindol-1-ylamine, la 3-imino-5-fluoro-3H-isoindol-1-ylamine, la 3-imino-5-chloro-3H-isoindol-1-ylamine, la 3-imino-5-méthylsulfanyl-3H-isoindol-1-ylamine, la 3-imino-5-méthoxy-3H-isoindol-1-ylamine, la 3-imino-5-éthoxy-3H-isoindol-1-ylamine, la 3-imino-5-propoxy-3H-isoindol-1-ylamine, la 3-imino-5-isopropoxy-3H-isoindol-1-ylamine, la 3-imino-5-butoxy-3H-isoindol-1-ylamine, la 3-imino-5-isobutoxy-3H-isoindol-1-ylamine, la 3-imino-5-terbutoxy-3H-isoindol-1-ylamine, la 3-imino-5-(2,2,2-trifluorométhyl)-3H-isoindol-1-ylamine, la 3-imino-5-(2,2,2-trifluoroéthoxy)-3H-isoindol-1-ylamine, la 3-imino-5-méthanesulfonyl-3H-isoindol-1-ylamine, la 3-imino-5,6-diméthyl-3H-isoindol-1-ylamine, la 3-imino-5,6-diéthyl-3H-isoindol-1-ylamine, la 3-imino-5,6-diméthoxy-3H-isoindol-1-ylamine, la 3-imino-5,6-diéthoxy-3H-isoindol-1-ylamine, la 3-imino-5,6-dibutoxy-3H-isoindol-1-ylamine, la 3-imino-5,6-bis-trifluorométhyl-3H-isoindol-1-ylamine, la 3-imino-5,6-dichloro-3H-isoindol-1-ylamine, la 5,6-bis-éthoxyméthyl-3-imino-3H-isoindol-1-ylamine, la 3-amino-1-imino-1H-isoindol-4,7-diol, la 4,7-dichloro-3-imino-3H-isoindol-1-ylamine, la 4,5,7-trichloro-3-imino-N6,N6-diméthyl-3H-isoindol-1,6-diamine, la 4,5,6,7-tétrachloro-3-imino-3H-isoindol-1-ylamine, la 4,5,6,7-tétrafluoro-3-imino-3H-isoindol-1-ylamine, la 3-butylimino-3H-isoindol-1-ylamine, la 2-(3-amino-isoindol-1-ylidèneamino)-éthanol, la 3-(3-amino-isoindol-1-ylidèneamino)-3-méthyl-pentane-1,5-diol, la N-(3-amino-isoindol-1-ylidène)-guanidine, la 7-imino-7H-pyrrolo[3,4-b]pyridin-5-ylamine, la 7-imino-7H-pyrrolo[3,4-b]pyrazin-5-ylamine, la 7-imino-2,3-diméthyl-7H-pyrrolo[3,4-b]pyrazin-5-ylamine, la 7-imino-7H-[1,4]dithiino[2,3-c] pyrrol-5-ylamine, la 7-imino-2,3-diméthyl-7H-[1,4]dithiino[2,3-c] pyrrol-5-ylamine, la 7-imino-2,3-dihydro-7H-[1,4]dithiino[2,3-c]pyrrol-5-ylamine, la 7-imino-2-méthyl-2,3-dihydro-7H-[1,4]dithiino [2,3-c]pyrrol-5-ylamine, la 3-amino-isoindol-1-one, la 3-amino-7-méthyl-isoindol-1-one, la 3-amino-7-hydroxyméthyl-isoindol-1-one, la 3-amino-7-chloro-isoindol-1-one, la 3-amino-4-chloro-isoindol-1-one, l'acide 3-amino-1-oxo-1H-isoindole-4-sulfonique, la 3-amino-4-nitro-isoindol-1-one, la 3-amino-6-nitro-isoindol-1-one, la 3-amino-6-méthyl-isoindol-1-one, la 3-amino-6-chloro-isoindol-1-one, la 3-amino-6-bromo-isoindol-1-one, la 3-amino-6-méthylsulfanyl-isoindol-1-one, la 3-amino-6-méthoxy-isoindol-1-one, la 3-amino-5-chloro-isoindol-1-one, la 3-amino-5-fluoro-isoindol-1-one, la 3-amino-5-méthoxy-isoindol-1-one, la 3-amino-5-nitro-isoindol-1-one, l'ester éthylique de l'acide 3-amino-1-oxo-1H-isoindole-5-carboxylique, la 3-amino-5,6-dichloro-isoindol-1-one, la 3-amino-5,6-dibromo-isoindol-1-one, la 3-amino-4,7-dichloro-isoindol-1-one, la 3-amino-4,5,7-trichloro-isoindol-1-one, la 3-amino-4,5,6,7-tétrachloro-isoindol-1-one, la 3-amino-4,5,7-trichloro-6-méthylsulfanyl-isoindol-1-one, la 3-amino-4,5,6,7-tétrabromo-isoindol-1-one, la 3-amino-4,5,6,7-tétrafluoro-isoindol-1-one, la 3-méthylamino-isoindol-1-one, la 3-éthylamino-isoindol-1-one, la 3-propylamino-isoindol-1-one, la 3-diméthylamino-isoindol-1-one, la 7-éthylamino-pyrrolo[3,4-b]pyridin-5-one, la 7-amino-pyrrolo[3,4-b]pyridin-5-one, la 3-amino-pyrrolo[3,4-c]pyridin-5-one, la 3-amino-6-méthyl-pyrrolo[3,4-c]pyridin-1-one, la 5-amino-pyrrolo[3,4-b]pyridin-7-one, la 7-amino-pyrrolo[3,4-b]pyrazin-5-one, la 7-amino-2-méthyl-pyrrolo[3,4-b]pyrazin-5-one, la 7-amino-2,3-diméthyl-pyrrolo[3,4-b]pyrazin-5-one, la 7-amino-2,3-dihydro-[1,4]dithiino[2,3-c]pyrrol-5-one, la 3-imino-2-méthyl-2,3-dihydro-isoindol-1-one, la 3-imino-2-éthyl-2,3-dihydro-isoindol-1-one, la 3-imino-2-propyl-2,3-dihydro-isoindol-1-one, la 2-hydroxyméthyl-3-imino-2,3-dihydro-isoindol-1-one, la 2-(2-hydroxyéthyl)-3-imino-2,3-dihydro-isoindol-1-one, l'acide 2-(1-imino-3-oxo-1,3-dihydro-isoindol-2-yl)-éthane sulfonique, l'acide 3-(1-imino-3-oxo-1,3-dihydro-isoindol-2-yl)-propionique, la 2-(3-hydroxypropyl)-3-imino-2,3-dihydro-isoindol-1-one et la 5-imino-6-méthyl-5,6-dihydro-pyrrolo[3,4-b]pyridin-7-one.

Dans le cadre de la présente invention:

Les atomes d'halogène désignent préférentiellement un atome de fluor, de chlore, de bromure ou d'iode.

Les radicaux alkyle, monohydroxyalkyle, polyhydroxyalkyles, alkylhydroxyalkyle, alkylesulfonyle, carboxyalkyle, aminoalkyle, alkylaminoalkyle, dihydroxyaminoalkyle peuvent être linéaires ou ramifiés.

Les groupements alkyle désignent notamment les groupements de 1 à 20 atomes de carbone, comme par exemple, les groupements méthyle, éthyle, propyle, isopropyle, n-propyle, butyle, n-butyle, tert-butyle, pentyle, n-pentyle, isopentyle, n-hexyle, isohexyle, heptyle, octyle, nonyle, decyle, undecyle, dodecyle et pentadecyle. Préférentiellement, les groupements alkyle désignent un groupement de 1 à 6 atomes de carbone;
ces groupements alkyle peuvent être substitués; par exemple, par un atome d'halogène, un radical cyano ou hydroxy, et peuvent ainsi représenter les radicaux trifluorométhyle, δ-chloropropyle, β-cyanoéthyle ou β-hydroxyéthyle.

Parmi les groupements monohydroxyalkyle, on peut notamment citer les groupements hydroxyméthyle, hydroxyéthyle, hydroxypropyle et hydroxybutyle.

Parmi les radicaux polyhydroxyalkyle, on peut par exemple citer les radicaux dihydroxyéthyle, dihydroxypropyle, trihydroxypropyle et dihydroxybutyle.

Les groupements alcoxy désignent un groupement -O-R, R représentant un groupement alkyle tel que défini ci-dessus.

Les groupements alcényles désignent un radical monovalent correspondant aux carbones éthyléniques, tels que, par exemple, alkyle ou 3,3diméthylallyle.

Les groupements acétyloxy désignent un groupement -O-CO-R, R représentant un groupement alkyle tel que défini ci-dessus.

Parmi les radicaux cycloalkyle, on peut notamment citer le cyclohexyle et le cyclopentyle.

Parmi les radicaux aryle, qui peuvent être mono ou polycycliques, on peut notamment citer les groupements phényle ou naphtyle.

Parmi les hétérocycles, qui peuvent être mono ou polycycliques et contenant un ou plusieurs hétéroatomes, on peut citer les cycles thiophène, pyrrole, imidazole, pyrazole, triazole, thiazole, furane, benzofurane, benzimidazole, benzothiazole, pyridyle, benzoxazole, quinolyle, quinazoyle, quinoxalyle ou naphtyle.

Parmi les radicaux alkylaryle, on peut notamment citer le groupement benzyle, phénéthyle ou naphthylméthyle.

Les groupements aminoaryle désignent les groupements NHR, R représentant un radical aryle.

Dans le cadre de la présente invention, les radicaux cycloakyles, aryle et les hétérocycles peuvent être substitués ou polysubstitués par exemple par un halogène, par un alkyle en C₁-C₄, un alkoxy en C₁-C₄, un groupe nitro, un groupe hydroxy, un groupe carboxylique, un groupe acétyloxy en C₁-C₄, un groupe carboxamide, un groupe sulfonamide, sulfonique, nitrile, -CF₃, en -OCF₃, ou par un radical cycloalkyle ou aryle pouvant être être substitué par un alkyle en C₁-C₄.

Dans le cadre de la présente invention, les formules (I) à (XXIII) ne sont pas limitées à celles spécifiquement décrites mais comprennent aussi leurs formes tautomères quand elles existent.

Au sens de la présente invention, les sels cosmétiquement acceptables des composés précités peuvent être des chlorhydrates, des sulfates, des bromhydrates ou des tartrates.

Les compositions de teinture des fibres kératiniques, en particulier des fibres kératiniques humaines telles que les cheveux, conformes à la présente invention sont essentiellement caractérisées par le fait qu'elles comprennent au moins un composé à méthylène actif tel que défini ci-dessus et au moins un composé choisi parmi un aldhéhyde de formule (XVIII), une cétone de formule (XIX) ou (XX), une quinone et un dérivé de la di-imino-isoindoline ou de la 3-amino-isoindolone tel que défini ci-dessus, dans un milieu approprié pour la teinture.

Préférentiellement, le composé à méthylène actif de ces compositions est choisi parmi le benzofuran(2H)one, le benzoylacétonitrile, le 5-amino-2H-pyrazol-3-ol et le 4-imino-4,5-dihydro-thiazol-2-ylamine.

Dans une forme de réalisation préférée de l'invention, le composé choisi parmi un aldéhyde de formule (XVIII), une cétone de formule (XIX) ou (XX), une quinone et un dérivé de la di-imino-isoindoline ou de la 3-amino-isoindolone est choisi parmi la naphtoquinone, l'isatine, la N-méthylisatine, la 3-imino-3H-isoindol-1-ylaminé, la 4-diméthylaminobenzaldéhyde et la 4-diméthylaminonaphtaldéhyde.

Le composé à méthylène actif peut être présent dans une concentration allant de 0,01 à 10 %, et préférentiellement de 0,05 à 5 % en poids par rapport au poids total de la composition.

Le composé choisi parmi un aldéhyde de formule (XVIII), une cétone de formule (XIX) ou (XX), une quinone et un dérivé de la di-imino-isoindoline ou de la 3-amino-isoindolone peut être présent dans une concentration allant de 0,01 à 10 % et préférentiellement de 0,05 à 5 % en poids par rapport au poids total de la composition.

Le milieu approprié pour la teinture est de préférence un milieu aqueux constitué par de l'eau et/ou des solvants organiques acceptables sur le plan cosmétique, et plus particulièrement, des alcools tels que l'alcool éthylique, l'alcool isopropylique, l'alcool benzylique, et l'alcool phényléthylique, ou des glycols ou éthers de glycol tels que, par exemple, l'éthylèneglycol et ses éthers monométhylique, monoéthylique et monobutylique, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, dans des concentrations comprises entre environ 0,5 et 20% et, de préférence, entre environ 2 et 10% en poids par rapport au poids total de la composition.

On peut également ajouter à la composition selon l'invention des amides gras tels que les mono- et di-éthanolamides des acides dérivés du coprah, de l'acide laurique ou de l'acide oléïque, à des concentrations comprises entre environ 0,05 et 10% en poids.

On peut encore ajouter à la composition selon l'invention des agents tensio-actifs bien connus de l'état de la technique et de type anionique, cationique, non-ionique, amphotère, zwittérionique ou leurs mélanges, de préférence en une proportion comprise entre environ 0,1 et 50% en poids et avantageusement entre environ 1 et 20% en poids par rapport au poids total de la composition.

On peut également utiliser des agents épaississants dans une proportion allant d'environ 0,2 à 20%.

Ladite composition tinctoriale peut contenir en outre divers adjuvants usuels tels que des agents anti-oxydants, des parfums, des agents séquestrants, des agents dispersants, des agents de conditionnement du cheveu, des agents conservateurs, des agents opacifiants, ainsi que tout autre adjuvant utilisé habituellement en teinture des matières kératiniques.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition tinctoriale selon l'invention peut être formulée à pH acide, neutre ou alcalin, le pH pouvant varier par exemple de 2 à 11 et de préférence de 5 à 10, et pouvant être ajusté au moyen d'agents d'alcalinisation ou d'agents d'acidification ou de tampons antérieurement bien connus.

Comme agents alcalinisants, on peut citer l'ammoniaque, les carbonates alcalins, les alcanolamines, par exemple les mono- di- et tri- éthanolamines et leurs dérivés, les hydroxydes de sodium ou de potassium, et les composés de formule : 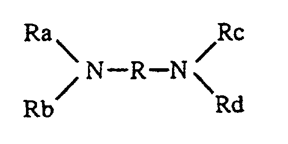 dans laquelle, R est un reste propylène éventuellement substitué par un groupement hydroxyle ou un radical alkyle en C₁-C₄; Ra, Rb, Rc et Rd, simultanément ou indépendamment l'un de l'autre représentent un atome d'hydrogène, un radical alkyle en C₁-C₄ ou hydroxyalkyle en C₁-C₄.

Les agents acidifiants sont classiquement des acides minéraux ou organiques comme par exemple les acides chlorhydriques, tartrique, citrique et phosphorique.

Parmi les tampons, on peut citer par exemple, le phosphate diacide de potassium/hydroxyde de sodium.

La composition appliquée sur les cheveux peut se présenter sous des formes diverses, telles que sous forme de liquide, de crème, de gel ou sous toute autre forme appropriée pour réaliser une teinture des fibres kératiniques. En particulier, elle peut être conditionnée sous pression en flacon aérosol en présence d'un agent propulseur et former une mousse.

Conformément à la présente invention, le procédé de teinture des fibres kératiniques, en particulier des fibres kératiniques humaines telles que les cheveux, est essentiellement caractérisé par le fait que l'on applique sur lesdites fibres un composant (A) constitué d'une composition renfermant dans un milieu approprié pour la teinture, au moins un composé à méthylène actif tel que défini ci-dessus, et un composant (B) constitué d'une composition contenant dans un milieu approprié pour la teinture, au moins un composé choisi parmi un aldehyde de formule (XVIII), une cétone de formule (XIX) ou (XX), une quinone et un dérivé de la di-imino-isoindoline ou de la 3-amino-isoindolone tel que, par exemple, un de ceux définis ci-dessus, de façon à permettre le développement d'une teinture sur lesdites fibres kératiniques.

Dans une forme de réalisation préférée du procédé de l'invention, les composants (A) et (B) sont mélangés juste avant emploi, puis la composition résultante est immédiatement appliquée sur les fibres kératiniques, et laissée agir pendant 1 à 60 minutes et préférentiellement de 1 à 30 minutes ; les fibres kératiniques étant ensuite rincées, lavées au shampooing, rincées à nouveau, puis séchées.

Un autre procédé de la présente invention consiste essentiellement à appliquer sur les fibres kératiniques le composant (A), suivi ou précédé de l'application sur lesdites fibres du composant (B), à laisser agir chaque composant pendant 1 à 60 minutes et préférentiellement de 1 à 30 minutes, à procéder éventuellement au rinçage à l'eau entre chaque application; les fibres kératiniques étant ensuite rincées, lavées au shampooing, rincées à nouveau, puis séchées.

Un objet de l'invention est aussi constitué par un agent de teinture pour les fibres kératiniques, en particulier des cheveux humains, caractérisé par le fait qu'il est constitué par les composants (A) et (B) stockés sous forme séparée, tels que définis ci-dessus.

Les composants (A) et (B) sont destinés, soit à être mélangés tous juste avant emploi, soit à être appliqués de façon successive sur les fibres à traiter.

Selon une forme de réalisation, on peut conditionner les différents composants (A) et (B) dans un dispositif à plusieurs compartiments encore appelé "kit de teinture" comportant tous les composants destinés à être appliqués pour une même teinture sur les fibres kératiniques, en particulier les fibres kératiniques humaines telles que les cheveux, en applications successives avec ou sans prémélange.

De tels dispositifs peuvent comporter un premier compartiment contenant le composant (A) renfermant la composé à méthylène actif et un second compartiment comportant le composant (B) renfermant le composé choisi parmi un aldéhyde, une cétone, une quinone et un dérivé de la di-imino-isoindoline ou de la 3-amino-isoindolone.

Une autre variante peut également consister à stocker le composant (A) ou le composant (B) sous forme d'une composiition anhydre et à prévoir un troisième compartiment contenant un milieu aqueux approprié pour la teinture et cosmétiquement acceptable. Dans ce cas, on mélange tout juste avant l'emploi le contenu du troisième compartiment dans l'un ou l'autre ou les deux compartiments contenant les composants anhydres (A) et (B) ou alors on mélange avant emploi les trois compartiments.
Des exemples concrets illustrant l'invention vont maintenant être donnés.

### EXEMPLES

### Exemple 1

On a préparé juste avant emploi la composition de teinture suivante:

| | |
|---|---|
| 3-imino-3H-isoindol-1-ylamine | 0,435 g |
| benzofuran-(2H)-one | 0,402 g |
| alcool éthylique | 30,0 g |
| eau qsp | 100 g |

On a appliqué la composition ci-dessus sur des mèches de cheveux gris naturels à 90% de blancs et on a laissé poser pendant 30 minutes. Après rinçage à l'eau courante et séchage, les cheveux ont été teints dans une nuance jaune citron.

### Exemple 2

On a préparé juste avant emploi la composition de teinture suivante:

| | |
|---|---|
| 4-diméthylaminobenzaldéhyde | 0,447 g |
| benzofuran-(2H)-one | 0,402 g |
| alcool éthylique | 30,0 g |
| eau q.s.p. | 100, g |

On a appliqué la composition ci-dessus sur des mèches de cheveux gris permanentés à 90% de blancs et on a laissé poser pendant 30 minutes. Après rinçage à l'eau courante et séchage, les cheveux ont été teints dans une nuance orange vif.

### Exemple 3

On a préparé juste avant emploi la composition de teinture suivante:

| | |
|---|---|
| 1,4-naphtoquinone | 0,447 g |
| benzofuran-(2H)-one | 0,402 g |
| alcool éthylique | 30,0 g |
| eau q.s.p. | 100, g |

On a appliqué la composition ci-dessus sur des mèches de cheveux gris permanentés à 90% de blancs et on a laissé poser pendant 30 minutes. Après rinçage à l'eau courante et séchage, les cheveux ont été teints dans une nuance violet foncé.

### Exemple 4

On a préparé juste avant emploi la composition de teinture suivante:

| | |
|---|---|
| isatine | 0,441 g |
| benzofuran-(2H)-one | 0,402 g |
| alcool éthylique | 30,0 g |
| eau q.s.p. | 100, g |

On a appliqué la composition ci-dessus sur des mèches de cheveux décolorés et on a laissé poser pendant 30 minutes. Après rinçage à l'eau courante et séchage, les cheveux ont été teints dans une nuance cuivré.

### Exemple 5

On a préparé juste avant emploi la composition de teinture suivante:

| | |
|---|---|
| 3-imino-3H-isoindol-1-ylamine | 0,435 g |
| benzoylacétonitrile | 0,435 g |
| alcool éthylique | 30,0 g |
| eau q.s.p. | 100, g |

On a appliqué la composition ci-dessus sur des mèches de cheveux décolorés et on a laissé poser pendant 30 minutes. Après rinçage à l'eau courante et séchage, les cheveux ont été teints dans une nuance jaune moutarde.

### Exemple 6

On a préparé juste avant emploi la composition de teinture suivante:

| | |
|---|---|
| 4-diméthylaminobenzaldéhyde | 0,447 g |
| benzoylacétonitrile | 0,402 g |
| alcool éthylique | 30,0 g |
| eau q.s.p. | 100, g |

On a appliqué la composition ci-dessus sur des mèches de cheveux gris permanentés à 90% et on a laissé poser pendant 30 minutes. Après rinçage à l'eau courante et séchage, les cheveux ont été teints dans une nuance orange rouge vif.

### Exemple 7

On a préparé juste avant emploi la composition de teinture suivante:

| | |
|---|---|
| 1,4-naphtoquinone | 0,474 g |
| benzoylacétonitrile | 0,435 g |
| alcool éthylique | 30,0 g |
| eau q.s.p. | 100, g |

On a appliqué la composition ci-dessus sur des mèches de cheveux gris permanentés à 90% et on a laissé poser pendant 30 minutes. Après rinçage à l'eau courante et séchage, les cheveux ont été teints dans une nuance noisette.

### Exemple 8

On a préparé juste avant emploi la composition de teinture suivante:

| | |
|---|---|
| isatine | 0,441 g |
| benzoylacétonitrile | 0,435 g |
| alcool éthylique | 30,0 g |
| eau q.s.p. | 100, g |

On a appliqué la composition ci-dessus sur des mèches de cheveux décolorés et on a laissé poser pendant 30 minutes. Après rinçage à l'eau courante et séchage, les cheveux ont été teints dans une nuance cuivré rouge.

### Exemple 9

On a préparé juste avant emploi la composition de teinture suivante:

| | |
|---|---|
| 3-imino-3H-isoindol-1-ylamine | 0,435 g |
| 6-hydroxy-benzofuran-(2H)-one | 0,450 g |
| alcool éthylique | 30,0 g |
| eau qsp | 100 g |

On a appliqué la composition ci-dessus sur des mèches de cheveux gris permanentés à 90% de blancs et on a laisé poser pendant 30 minutes. Après rinçage à l'eau courante et séchage, les cheveux ont été teints dans une nuance jaune doré.

### Exemple 10

On a préparé juse avant emploi la composition de teinure suivante:

| | |
|---|---|
| 4-diméthylaminobenzaldéhyde | 0,447 g |
| 6-hydroxy-benzofuran-(2H)-one | 0,450 g |
| alcool éthylique | 30,0 g |
| eau qsp | 100 g |

On a appliqué la composition ci-dessus sur des mèches de cheveux gris permanentés à 90% de blancs et on a laissé poser pendant 30 minutes. Après rinçage à l'eau courante et séchage, les cheveux ont été teints dans une nuance jaune orangé.

### Exemple 11

On a préparé juste avant emploi la composition de teinture suivante:

| | |
|---|---|
| 1,4-naphtoquinone | 0,474 g |
| 6-hydroxy-benzofuran-(2H)-one | 0,450 g |
| alcool éthylique | 30,0 g |
| eau qsp | 100 g |

On a appliqué la composition ci-dessus sur des mèches de cheveux gris permanentés à 90% de blancs et on a laissé poser pendant 30 minutes. Après rinçage à l'eau courante et séchage, les cheveux ont été teints dans une nuance noisette.

### Exemple 12

On a préparé juste avant emploi la composition de teinture suivante:

| | |
|---|---|
| isatine | 0,441 g |
| 6-hydroxy-benzofuran-(2H)-one | 0,450 g |
| alcool éthylique | 30,0 g |
| eau qsp | 100 g |

On a appliqué la composition ci-dessus sur des mèches de cheveux gris permanentés à 90% de blancs et on a laissé poser pendant 30 minutes. Après rinçage à l'eau courante et séchage, les cheveux ont été teints dans une nuance jaune légèrement cuivré.

### Exemple 13

On a préparé juste avant emploi la composition de teinture suivante:

| | |
|---|---|
| 3-imino-3H-isoindol-1-ylamine | 0,435 g |
| 5-amino-2H-pyrazol-3-ol | 0,297 g |
| alcool éthylique | 30,0 g |
| eau qsp | 100 g |

On a appliqué la composition ci-dessus sur des mèches de cheveux décolorés et on a laissé poser pendant 30 minutes. Après rinçage à l'eau courante et séchage, les cheveux ont été teints dans une nuance beige doré.

### Exemple 14

On a préparé juste avant emploi la composition de teinture suivante:

| | |
|---|---|
| 4-diméthylaminobenzaldéhyde | 0,447 g |
| 5-amino-2H-pyrazol-3-ol | 0,297 g |
| alcool éthylique | 30,0 g |
| eau qsp | 100 g |

On a appliqué la composition ci-dessus sur des mèches de cheveux gris permanentés à 90% de blancs et on a laissé poser pendant 30 minutes. Après rinçage à l'eau courante et séchage, les cheveux ont été teints dans une nuance jaune orangé.

### Exemple 15

On a préparé juste avant emploi la composition de teinture suivante:

| | |
|---|---|
| 1,4-naphtoquinone | 0,474 g |
| 5-amino-2H-pyrazol-3-ol | 0,297 g |
| alcool éthylique | 30,0 g |
| eau qsp | 100 g |

On a appliqué la composition ci-dessus sur des mèches de cheveux gris permanentés à 90% de blancs et on a laissé poser pendant 30 minutes. Après rinçage à l'eau courante et séchage, les cheveux ont été teints dans une nuance marron doré.

### Exemple 16

On a préparé juste avant emploi la composition de teinture suivante:

| | |
|---|---|
| 3-imino-3H-isoindol-1-ylamine | 0,435 g |
| 3-amino-1-phényl-2-pyrazolin-5-one | 0,525 g |
| alcool éthylique | 30,0 g |
| eau qsp | 100 g |

On a appliqué la composition ci-dessus sur des mèches de naturels à 90% de blancs et on a laissé poser pendant 30 minutes. Après rinçage à l'eau courante et séchage, les cheveux ont été teints dans une nuance jaune vert.

### Exemple 17

On a préparé juste avant emploi la composition de teinture suivante:

| | |
|---|---|
| 4-diméthylaminobenzaldéhyde | 0,447 g |
| 3-amino-1-phényl-2-pyrazolin-5-one | 0,525 g |
| alcool éthylique | 30,0 g |
| eau qsp | 100 g |

On a appliqué la composition ci-dessus sur des mèches de cheveux décolorés on a laissé poser pendant 30 minutes. Après rinçage à l'eau courante et séchage, les cheveux ont été teints dans une nuance orange vif.

### Exemple 18

On a préparé juste avant emploi la composition de teinture suivante:

| | |
|---|---|
| 1,4-naphtoquinone | 0,474 g |
| 3-amino-1-phényl-2-pyrazolin-5-one | 0,525 g |
| alcool éthylique | 30,0 g |
| eau qsp | 100 g |

On a appliqué la composition ci-dessus sur des mèches de cheveux gris permanentés à 90% de blancs et on a laissé poser pendant 30 minutes. Après rinçage à l'eau courante et séchage, les cheveux ont été teints dans une nuance marron foncé.

### Exemple 19

On a préparé juste avant emploi la composition de teinture suivante:

| | |
|---|---|
| 3-imino-3H-isoindol-1-ylamine, Hcl | 0,435 g |
| 4-imino-4,5-dihydro-thiazol-2-ylamine | 0,454 g |
| alcool éthylique | 30,0 g |
| eau qsp | 100 g |

On a appliqué la composition ci-dessus sur des mèches de cheveux gris naturels à 90% de blancs et on a laissé poser pendant 30 minutes. Après rinçage à l'eau courante et séchage, les cheveux ont été teints dans une nuance orangé vif.

### Exemple 20

On a préparé juste avant emploi la composition de teinture suivante:

| | |
|---|---|
| 3-imino-3H-isoindol-1-one | 0,438 g |
| 4-imino-4,5-dihydro-thiazol-2-ylamine, Hcl | 0,454 g |
| alcool éthylique | 30,0 g |
| eau qsp | 100 g |

On a appliqué la composition ci-dessus sur des mèches de cheveux gris naturels à 90% de blancs et on a laissé poser pendant 30 minutes. Après rinçage à l'eau courante et séchage, les cheveux ont été teints dans une nuance jaune.

### Exemple 21

On a préparé juste avant emploi la composition de teinture suivante:

| | |
|---|---|
| 4-diméthylaminobenzaldéhyde | 0,447 g |
| 4-imino-4,5-dihydro-thiazol-2-ylamine, Hcl | 0,454 g |
| alcool éthylique | 30,0 g |
| eau qsp | 100 g |

## Revendications

1. Utilisation, pour la teinture des fibres kératiniques, d'au moins un composé à méthylène actif comportant un groupement méthylène substitué par deux groupements à effet electro- ou mésomère attracteur choisi parmi :
1) les composés de formule (I) suivante: 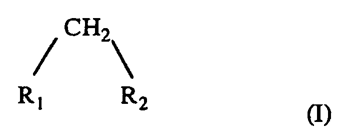 dans laquelle :
R₁ désigne un groupement -COR ou -COOR avec R désignant un atome d'hydrogène ou un groupement alkyle,
R₂ désigne les groupements désignés par R₁, un groupement nitrile, un groupement aryle non substitué ou substitué par un halogène, par un alkyle en C₁-C₄, un alkoxy en C₁-C₄, un groupe nitro, un groupe hydroxy, un groupe carboxylique, un groupe acétyloxy en C₁-C₄, un groupe carboxamide, un groupe sulfonamide, sulfonique, nitrile, -CF₃, en -OCF₃, ou par un radical cycloalkyle ou aryle pouvant être substitué par un alkyle en C₁-C₄ ;
un groupement alkylaryle, l'alkyle étant non substitué ou substitué par un atome d'halogène, un radical cyano ou hydroxy et l'aryle étant non substitué ou substitué par un halogène, par un alkyle en C₁-C₄, un alkoxy en C₁-C₄, un groupe nitro, un groupe hydroxy, un groupe carboxylique, un groupe acétyloxy en C₁-C₄, un groupe carboxamide, un groupe sulfonamide, sulfonique, nitrile, -CF₃, en -OCF₃, ou par un radical cycloalkyle ou aryle pouvant être substitué par un alkyle en C₁-C₄ ;
ou un hétérocycle non substitué ou substitué par un halogène, par un alkyle en C₁-C₄, un alkoxy en C₁-C₄, un groupe nitro, un groupe hydroxy, un groupe carboxylique, un groupe acétyloxy en C₁-C₄, un groupe carboxamide, un groupe sulfonamide, sulfonique, nitrile, -CF₃, en -OCF₃, ou par un radical cycloalkyle ou aryle pouvant être substitué par un alkyle en C₁-C₄ ;
2) les composés de formule (II) suivante: 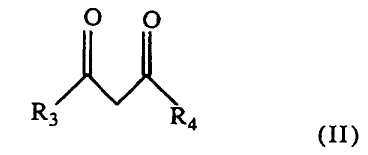 dans laquelle :
R₃ désigne les groupements désignés par R₂
R₄ désigne un groupement alkyle non substitué ou substitué par un atome d'halogène, un radical cyano ou hydroxy, pouvant représenter les radicaux trifluorométhyle, δ-chloropropyle, β-cyanoéthyle ou β-hydroxyéthyle ; un groupement acétyloxy ; un groupement cycloalkyle ;
un groupement alkylaryle, l'alkyle étant non substitué ou substitué par un atome d'halogène, un radical cyano ou hydroxy et l'aryle étant non substitué ou substitué par un halogène, par un alkyle en C₁-C₄, un alkoxy en C₁-C₄, un groupe nitro, un groupe hydroxy, un groupe carboxylique, un groupe acétyloxy en C₁-C₄, un groupe carboxamide, un groupe sulfonamide, sulfonique, nitrile, -CF₃, en -OCF₃, ou par un radical cycloalkyle ou aryle pouvant être substitué par un alkyle en C₁-C₄ ;
un groupement aralkyle ; un groupement aryle non substitué ou substitué par un halogène, par un alkyle en C₁-C₄, un alkoxy en C₁-C₄, un groupe nitro, un groupe hydroxy, un groupe carboxylique, un groupe acétyloxy en C₁-C₄, un groupe carboxamide, un groupe sulfonamide, sulfonique, nitrile, -CF₃, en -OCF₃, ou par un radical cycloalkyle ou aryle pouvant être substitué par un alkyle en C₁-C₄ ;
un groupement aminoaryle non substitué ou substitué par un halogène, par un alkyle en C₁-C₄, un alkoxy en C₁-C₄, un groupe nitro, un groupe hydroxy, un groupe carboxylique, un groupe acétyloxy en C₁-C₄, un groupe carboxamide, un groupe sulfonamide, sulfonique, nitrile, -CF₃, en -OCF₃, ou par un radical cycloalkyle ou aryle pouvant être substitué par un alkyle en C₁-C₄ ;
ou un hétérocycle non substitué ou substitué par un halogène, par un alkyle en C₁-C₄, un alkoxy en C₁-C₄, un groupe nitro, un groupe hydroxy, un groupe carboxylique, un groupe acétyloxy en C₁-C₄, un groupe carboxamide, un groupe sulfonamide, sulfonique, nitrile, -CF₃, en -OCF₃, ou par un radical cycloalkyle ou aryle pouvant être substitué par un alkyle en C₁-C₄ ;
3) les composés de formule (III) suivante : 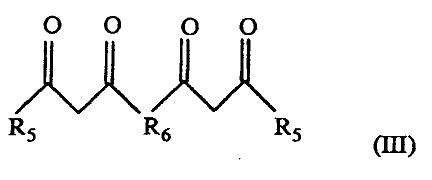 dans laquelle :
R₅ désigne les groupements désignés par R₂
R₆ désigne un groupement aryle non substitué ou substitué par un halogène, par un alkyle en C₁-C₄, un alkoxy en C₁-C₄, un groupe nitro, un groupe hydroxy, un groupe carboxylique, un groupe acétyloxy en C₁-C₄, un groupe carboxamide, un groupe sulfonamide, sulfonique, nitrile, -CF₃, en -OCF₃, ou par un radical cycloalkyle ou aryle pouvant être substitué par un alkyle en C₁-C₄ ;
un groupement aralkyle, l'alkyle étant non substitué ou substitué par un atome d'halogène, un radical cyano ou hydroxy et l'aryle étant non substitué ou substitué par un halogène, par un alkyle en C₁-C₄, un alkoxy en C₁-C₄, un groupe nitro, un groupe hydroxy, un groupe carboxylique, un groupe acétyloxy en C₁-C₄, un groupe carboxamide, un groupe sulfonamide, sulfonique, nitrile, -CF₃, en -OCF₃, ou par un radical cycloalkyle ou aryle pouvant être substitué par un alkyle en C₁-C₄ ;
un groupement aminoaryle non substitué ou substitué par un halogène, par un alkyle en C₁-C₄, un alkoxy en C₁-C₄, un groupe nitro, un groupe hydroxy, un groupe carboxylique, un groupe acétyloxy en C₁-C₄, un groupe carboxamide, un groupe sulfonamide, sulfonique, nitrile, -CF₃, en -OCF₃, ou par un radical cycloalkyle ou aryle pouvant être substitué par un alkyle en C₁-C₄ ;
ou un hétérocycle non substitué ou substitué par un halogène, par un alkyle en C₁-C₄, un alkoxy en C₁-C₄, un groupe nitro, un groupe hydroxy, un groupe carboxylique, un groupe acétyloxy en C₁-C₄, un groupe carboxamide, un groupe sulfonamide, sulfonique, nitrile, -CF₃, en -OCF₃, ou par un radical cycloalkyle ou aryle pouvant être substitué par un alkyle en C₁-C₄ ;
4) les dérivés de pyrazole (i) de formules (IV) et (V) suivantes : 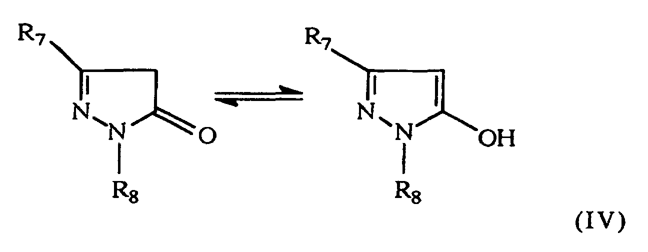 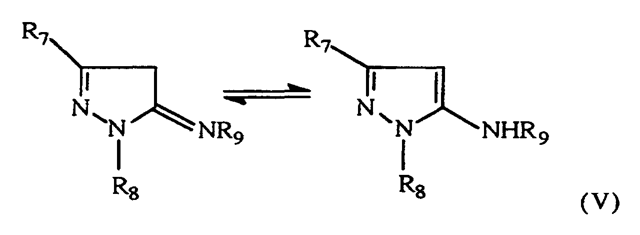 dans lesquelles:
R₇ et R₈, identiques ou différents, désignent les groupements désignés par R₄,
R₉ désigne un atome d'hydrogène ou un groupement alkyle non substitué ou substitué par un atome d'halogène, un radical cyano ou hydroxy, pouvant représenter les radicaux trifluorométhyle, δ-chloropropyle, β-cyanoéthyle ou β-hydroxyéthyle;
et (ii) formés de deux cycles pyrazole de formule (IV) ou (V) reliés par R₇ ou R₈;
5) les dérivés d'acides barbituriques (i) de formule (VI) suivante: 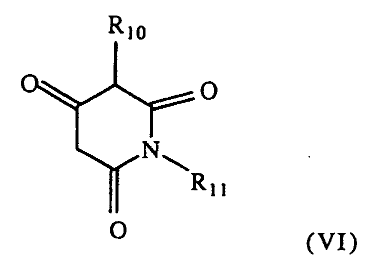 dans laquelle:
R₁₀ et R₁₁, identiques ou différents, désignent un groupement alkyle non substitué ou substitué par un atome d'halogène, un radical cyano ou hydroxy, pouvant représenter les radicaux trifluorométhyle, δ-chloropropyle, β-cyanoéthyle ou β-hydroxyéthyle ; un groupement alcényle ; un groupement cycloalkyle ; un groupement alkylaryle, l'alkyle étant non substitué ou substitué par un atome d'halogène, un radical cyano ou hydroxy et l'aryle étant non substitué ou substitué par un halogène, par un alkyle en C₁-C₄, un alkoxy en C₁-C₄, un groupe nitro, un groupe hydroxy, un groupe carboxylique, un groupe acétyloxy en C₁-C₄, un groupe carboxamide, un groupe sulfonamide, sulfonique, nitrile, -CF₃, en -OCF₃, ou par un radical cycloalkyle ou aryle pouvant être substitué par un alkyle en C₁-C₄ ;
ou un groupement aryle non substitué ou substitué par un halogène, par un alkyle en C₁-C₄, un alkoxy en C₁-C₄, un groupe nitro, un groupe hydroxy, un groupe carboxylique, un groupe acétyloxy en C₁-C₄, un groupe carboxamide, un groupe sulfonamide, sulfonique, nitrile, -CF₃, en -OCF₃, ou par un radical cycloalkyle ou aryle pouvant être substitué par un alkyle en C₁-C₄ ; et (ii) formés de deux cycles de formule (VI) reliés par R₁₀ ou R₁₁;
6) Les dérivés de pyridines de formule (VII) : 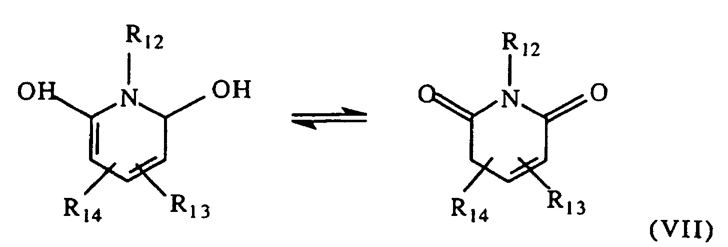 dans laquelle :
R₁₂ désigne un groupement alkyle non substitué ou substitué par un atome d'halogène, un radical cyano ou hydroxy, pouvant représenter les radicaux trifluorométhyle, δ-chloropropyle, β-cyanoéthyle ou β-hydroxyéthyle ; un groupement aryle non substitué ou substitué par un halogène, par un alkyle en C₁-C₄, un alkoxy en C₁-C₄, un groupe nitro, un groupe hydroxy, un groupe carboxylique, un groupe acétyloxy en C₁-C₄, un groupe carboxamide, un groupe sulfonamide, sulfonique, nitrile, -CF₃, en -OCF₃, ou par un radical cycloalkyle ou aryle pouvant être substitué par un alkyle en C₁-C₄ ;
R₁₃ désigne un atome d'hydrogène, un groupement alkyle non substitué ou substitué par un atome d'halogène, un radical cyano ou hydroxy, pouvant représenter les radicaux trifluorométhyle, δ-chloropropyle, β-cyanoéthyle ou β-hydroxyéthyle ; un groupement aryle non substitué ou substitué par un halogène, par un alkyle en C₁-C₄, un alkoxy en C₁-C₄, un groupe nitro, un groupe hydroxy, un groupe carboxylique, un groupe acétyloxy en C₁-C₄, un groupe carboxamide, un groupe sulfonamide, sulfonique, nitrile, -CF₃, en -OCF₃, ou par un radical, cycloalkyle ou aryle pouvant être substitué par un alkyle en C₁-C₄ ;
R₁₄ désigne un atome d'hydrogène, un groupement nitrile, un groupement alkyle non substitué ou substitué par un atome d'halogène, un radical cyano ou hydroxy, pouvant représenter les radicaux trifluorométhyle, δ-chloropropyle, β-cyanoéthyle ou β-hydroxyéthyle, un groupement COOR, R désignant un atome d'hydrogène ou un groupement alkyle non substitué ou substitué par un atome d'halogène, un radical cyano ou hydroxy, pouvant représenter les radicaux trifluorométhyle, δ-chloropropyle, β-cyanoéthyle ou β-hydroxyéthyle;
7) les dérivés de formule (VIII) suivante : 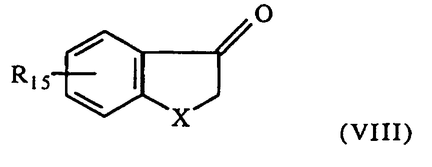 dans laquelle :
X désigne un atome d'oxygène, de soufre, d'azote ou un groupement NR', R' désignant un groupement alkyle,
R₁₅ désigne un atome d'hydrogène, de chlore, de brome, un groupement hydroxy, nitro, alkyle, alcoxy, carboxamide, sulfonamide ou nitrile ;
8) les dérivés de formules (IX) et (X) suivantes: 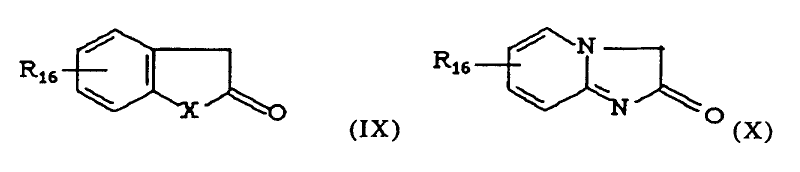 dans lesquelles :
X désigne un atome d'oxygène, de soufre, d'azote, ou un groupement NR', R' désignant un groupement alkyle,
R₁₆ désigne les atomes et groupements désignés par R₁₅ ;
9) les dérivés de formule (XI) suivante : 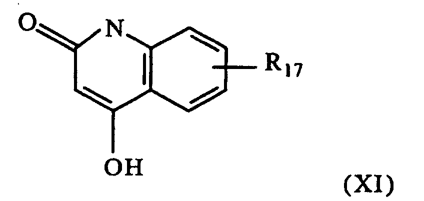 dans laquelle :
R₁₇ désigne un atome d'hydrogène, un groupement hydroxy ou un groupement alkyle non substitué ou substitué par un atome d'halogène, un radical cyano ou hydroxy, pouvant représenter les radicaux trifluorométhyle, δ-chloropropyle, β-cyanoéthyle ou β-hydroxyéthyle; ou un groupement aryle non substitué ou substitué par un halogène, par un alkyle en C₁-C₄, un alkoxy en C₁-C₄, un groupe nitro, un groupe hydroxy, un groupe carboxylique, un groupe acétyloxy en C₁-C₄, un groupe carboxamide, un groupe sulfonamide, sulfonique, nitrile, -CF₃, en -OCF₃, ou par un radical cycloalkyle ou aryle pouvant être substitué par un alkyle en C₁-C₄ ; ou
un groupement alkylaryle, l'alkyle étant non substitué ou substitué par un atome d'halogène, un radical cyano ou hydroxy et l'aryle étant non substitué ou substitué par un halogène, par un alkyle en C₁-C₄, un alkoxy en C₁-C₄, un groupe nitro, un groupe hydroxy, un groupe carboxylique, un groupe acétyloxy en C₁-C₄, un groupe carboxamide, un groupe sulfonamide, sulfonique, nitrile, -CF₃, en -OCF₃, ou par un radical cycloalkyle ou aryle pouvant être substitué par un alkyle en C₁-C₄ ;
10) les dérivés d'indandione de formule (XII) suivante: 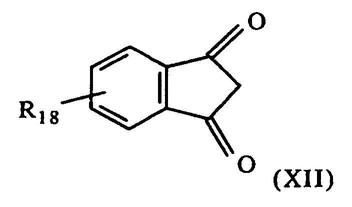 dans laquelle :
R₁₈ désigne un atome d'hydrogène, de chlore, de brome, un groupement nitro, alkyle, alkoxy, carboxamide, sulfonamide ou nitrile ;
11) les dérivés de formule (XIII) suivante : 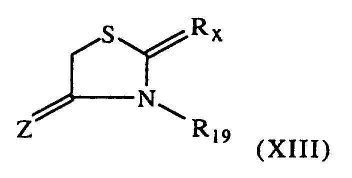 dans laquelle :
Z désigne O, ou NR avec R = H ou alkyle
Rₓ désigne un atome de soufre, ou NR, R désignant un atome d'hydrogène ou un groupement alkyle ;
R₁₉ désigne un atome d'hydrogène, un groupement alkyle, alcoxy, nitro ou nitrile ;
12) les dérivés de dioxopyrazole de formule (XIV) suivante: 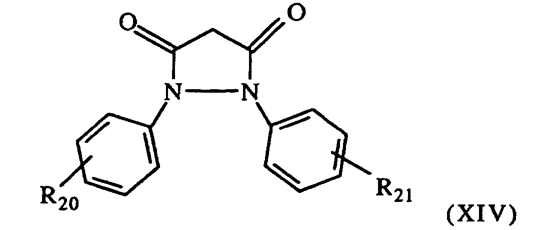 dans laquelle :
R₂₀ et R₂₁ identiques ou différents, désignent un atome d'hydrogène, un groupement alkyle, alcoxy, nitro ou nitrile ;
13) les dérivés de 5-oxoimidazole de formule (XV) suivante : 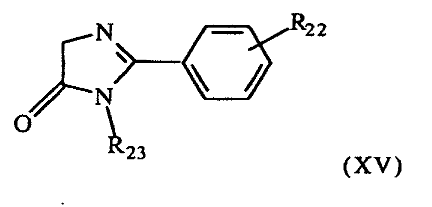 dans laquelle :
R₂₂ désigne un atome d'hydrogène ou un groupement alkyle
R₂₃ désigne un atome d'hydrogène, un groupement alkyle, alcoxy. nitro ou nitrile ;
14) les dérivés de déhydrobutyrolactone de formule (XVI) suivante: 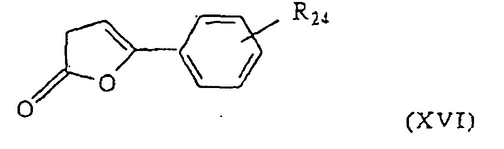 dans laquelle : R₂₄ désigne un atome d'hydrogène, un groupement alkyle, alcoxy, nitro ou nitrile ;
15) Les composés de formule (XVII) suivante : 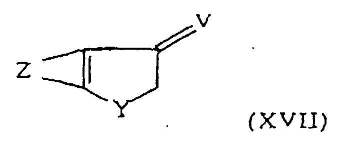 dans laquelle :
Z forme un cycle aromatique
V désigne un atome d'oxygène ou un groupement A-CH₂-E
dans lequel A ou E désigne un substituant ayant une constante de Hammet comprise entre 0,4 et 2,0 ou des substituants dont la somme des constantes de Hammet est comprise enre 0,4 et 2,0
Y désigne Co, O, S, NR, lorsque V est différent d'un atome d'oxygène ou désigne CS, C = NR₂, SO, SO₂, avec R₁ ou R₂ désignant un atome d'hydrogène ou un radical alkyle
et parmi les sels cosmétiquement acceptables des composés définis ci-dessus;
et d'au moins un composé choisi parmi :
- un aldéhyde correspond à la formule (XVIII) suivante: 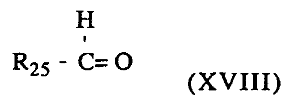 dans laquelle :
R₂₅ désigne un groupement de formule (XVIII A) suivante: 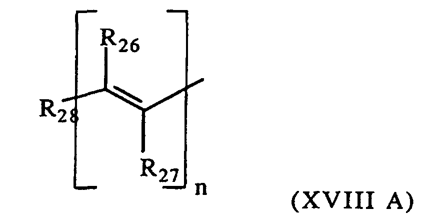
dans laquelle R₂₆ et R₂₇, identiques ou différents, désignent un atome d'hydrogène, un groupement alkyle, mono ou polyhydroxyalkyle, alkylhydroxyalkyle, alcoxy, -CF₃ ou -OCF₃,
R₂₆ et R₂₇ peuvent également former conjointement avec les atomes auxquels ils sont rattachés un cycle aryle ou un hétérocyclique à 5 ou 6 chaînons, lesdits cycles pouvant être non substitué ou substitué par un halogène, par un alkyle en C₁-C₄, un alkoxy en C₁-C₄, un groupe nitro, un groupe hydroxy, un groupe carboxylique, un groupe acétyloxy en C₁-C₄, un groupe carboxamide, un groupe sulfonamide, sulfonique, nitrile, -CF₃, en -OCF₃, ou par un radical cycloalkyle ou aryle pouvant être substitué par un alkyle en C₁-C₄ ;
n désigne un nombre entier de 0 à 3,
R₂₈ désigne les substituants désignés par R₂₆, un groupement aryle, alkylaryle, l'alkyle étant non substitué ou substitué par un atome d'halogène, un radical cyano ou hydroxy et l'aryle étant non substitué ou substitué par un halogène, par un alkyle en C₁-C₄, un alkoxy en C₁-C₄, un groupe nitro, un groupe hydroxy, un groupe carboxylique, un groupe acétyloxy en C₁-C₄, un groupe carboxamide, un groupe sulfonamide, sulfonique, nitrile, -CF₃, en -OCF₃, ou par un radical cycloalkyle ou aryle pouvant être substitué par un alkyle en C₁-C₄ ;
un groupe hétérocyclique à 5 ou 6 chaînons non substitué ou substitué par un halogène, par un alkyle en C₁-C₄, un alkoxy en C₁-C₄, un groupe nitro, un groupe hydroxy, un groupe carboxylique, un groupe acétyloxy en C₁-C₄, un groupe carboxamide, un groupe sulfonamide, sulfonique, nitrile, -CF₃, en -OCF₃, ou par un radical cycloalkyle ou aryle pouvant être substitué par un alkyle en C₁-C₄ ;
ou aux sels cosmétiquement acceptables de ces composés.
- une cétone correspond aux formules (XIX) ou (XX) suivantes: 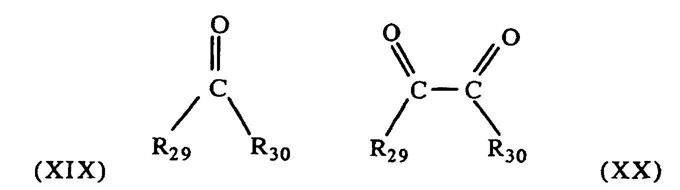 dans lesquelles :
R₂₉ désigne les substituants désignés par R₂₅
R₃₀ désigne un groupement alkyle, mono ou polyhydroxyalkyle, alkylhydroxyalkyle ; un groupement aryle, alkylaryle, un hétérocyclique à 5 ou 6 chaînons non substitué ou substitué par un halogène, par un alkyle en C₁-C₄, un alkoxy en C₁-C₄, un groupe nitro, un groupe hydroxy, un groupe carboxylique, un groupe acétyloxy en C₁-C₄, un groupe carboxamide, un groupe sulfonamide, sulfonique, nitrile, -CF₃, en -OCF₃, ou par un radical cycloalkyle ou aryle pouvant être substitué par un alkyle en C₁-C₄ ;
R₂₉ et R₃₀ peuvent également former conjointement avec les atomes auxquels ils sont rattachés un cycle aryle à 5 ou 6 chaînons, ou un hétérocyclique comprenant des hétéroatomes tels que N ou S, ledit cycle pouvant lui-même être rattaché à un cycle aryle à 5 ou 6 chaînons ou à un hétérocycle comprenant des hétéroatomes tels que N ou S, lesdits cycles pouvant être non substitué ou substitué par un halogène, par un alkyle en C₁-C₄, un alkoxy en C₁-C₄, un groupe nitro, un groupe hydroxy, un groupe carboxylique, un groupe acétyloxy en C₁-C₄, un groupe carboxamide, un groupe sulfonamide, sulfonique, nitrile, -CF₃, en -OCF₃, ou par un radical, cycloalkyle ou aryle pouvant être substitué par un alkyle en C₁-C₄ ; ou aux sels cosmétiquement acceptables de ces composés.
- une quinone correspondant aux formules (XXI) et (XXII) suivantes : 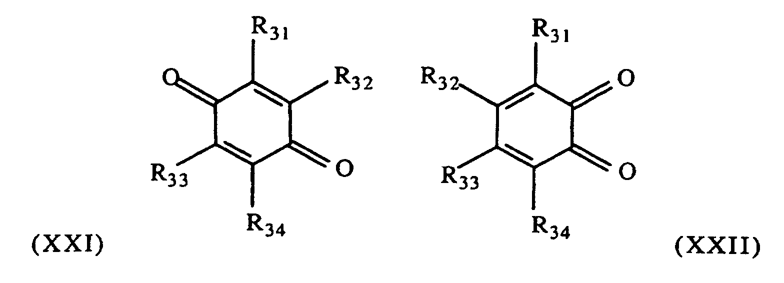 dans lesquelles : R₃₁ désigne un atome d'hydrogène, d'halogène, un groupement sulfonique ou alcoxy. R₃₂, R₃₃ et R₃₄, identiques ou différents désignent un atome d'hydrogène, d'halogène, un groupement hydroxy, alkyle, mono ou polyhydroxyalkyle, alkylhydroxyalkyle, alkylsulfonyle, carboxyalkyle, aminoalkyle, alkylaminoalkyle, (di-hydroxy)alkylaminoalkyle, ou alkyle-NR'R", avec R' et R" désignant alkyle ou pouvant former ensemble avec l'atome d'azote auxquels ils sont rattachés un cycle aryle ou un hétérocycle à 5 ou 6 chaînons, un groupement aryle, un groupe amino pouvant être substitué par un alkyle
ou un hydroxyalkyle, R₃₁ et R₃₂, R₃₁ et R₃₃ ou R₃₃ et R₃₄ peuvent former conjointement avec les atomes auxquels ils sont rattachés un cycle aryle
ou un hétérocycle à 5 ou 6 chaînons, non substitué ou substitué par un halogène, par un alkyle en C₁-C₄, un alkoxy en C₁-C₄, un groupe nitro, un groupe hydroxy, un groupe carboxylique, un groupe acétyloxy en C₁-C₄, un groupe carboxamide, un groupe sulfonamide, sulfonique, nitrile, -CF₃, en -OCF₃, ou par un radical cycloalkyle ou aryle pouvant être substitué par un alkyle en C₁-C₄ ;
ou aux sels cosmétiquement acceptables de ces composés,
et un dérivé de la di-imino-isoindoline, ou de la 3-amino-isoindolone correspondant à la formule (XXIII) suivante: 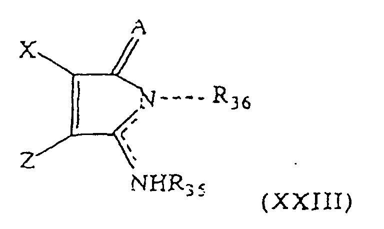 dans laquelle : R₃₅ et R₃₆, identiques ou différents, désignent un atome d'hydrogène, un groupement alkyle, mono ou polyhydroxyalkyle, alkylhydroxyalkyle, aminoalkyle, alkylaminoalkyle, (di-hydroxy)alkylaminoalkyle, ou un groupement alkyle NR'R", avec R' et R" désignant alkyle ou pouvant former conjointement avec l'atome d'azote auxquels ils sont rattachés un cycle aryle ou un hétérocycle à 5 ou 6 chaînons, A désigne un atome d'oxygène ou NH, X et Z forment ensemble un cycle aryle ou un hétérocycle à 5 ou 6 chaînons, non substitué ou substitué par un halogène, par un alkyle en C₁-C₄, un alkoxy en C₁-C₄, un groupe nitro, un groupe hydroxy, un groupe carboxylique, un groupe acétyloxy en C₁-C₄, un groupe carboxamide, un groupe sulfonamide, sulfonique, nitrile, -CF₃, en -OCF₃, ou par un radical cycloalkyle ou aryle pouvant être substitué par un alkyle en C₁-C₄ ;
ou aux sels cosmétiquement acceptables de ces composés,
ces composés permettant d'obtenir, par réaction sans agent oxydant, une coloration desdites fibres kératiniques, ainsi que les combinaisons des composés suivantes :
- 3-imino-3H-isoindol-1-ylamine et benzoylacétonitrile ;
- 4-diméthylaminobenzaldéhyde et benzoylacétonitrile ;
- 1,4-naphtoquinone et benzoylacétonitrile ;
- isatine et benzoylacétonitrile ;
- 3-imino-3H-isoindol-1-ylamine et 5-amino-2H-pyrazol-3-ol ;
- 4-diméthylaminobenzaldéhyde et 5-amino-2H-pyrazol-3-ol ;
- 1,4-naphtoquinone et 5-amino-2H-pyrazol-3-ol ;
- 3-imino-3H-isoindol-1-ylamine et 3-amino-1-phényl-2-pyrazolin-5-one ;
- 4-diméthylaminobenzaldéhyde et 3-amino-1-phényl-2-pyrazolin-5-one ;
- 1,4-naphtoquinone et 3-amino-1-phényl-2-pyrazolin-5-one.

2. Composition de teinture de fibres kératiniques, en particulier des fibres kératiniques humaines telles que les cheveux, **caractérisée par le fait qu'**elle comprend au moins un composé à méthylène actif choisi parmi les composés définis selon la revendication 1 et au moins un composé choisi parmi un aldéhyde, une cétone, une quinone et un dérivé de la di-imino-isoindoline ou de la 3-amino-isoindoline selon la revendication 1, dans un milieu approprié pour la teinture.

3. Composition de teinture selon la revendication 2, **caractérisée par le fait qu'**elle a un pH compris entre 2 et 11.

4. Composition selon la revendication 2 ou 3, **caractérisée par le fait que** le composé à méthylène actif est présent dans une concentration allant de 0,01 à 10 % et de préférence de 0,05 à 5 % en poids par rapport au poids total de la composition.

5. Composition selon l'une quelconque des revendications 2 à 4, **caractérisée par le fait que** le composé choisi parmi un aldéhyde, une cétone, une quinone et un dérivé de la di-imino-isoindoline ou de la 3-amino-isoindolone est présent dans une concentration allant de 0,01 à 10 % et de préférence de 0,05 à 5 % en poids par rapport au poids total de la composition.

6. Composition selon l'une quelconque des revendications 2 à 5, **caractérisée par le fait que** le milieu approprié pour la teinture est un milieu aqueux constitué par de l'eau et/ou des solvants organiques choisis parmi les alcools, les glycols et les éthers de glycol, dans des proportions comprises entre 0,5 et 20% en poids par rapport au poids total de la composition.

7. Procédé de teinture des fibres kératiniques, en particulier des fibres kératiniques humaines telles que les cheveux, **caractérisé par le fait qu'**il consiste à appliquer sur lesdites fibres un composant (A) constitué d'une composition renfermant dans un milieu approprié pour la teinture, au moins un composé à méthylène actif tel que défini dans la revendication 1 et au moins un composant (B) constitué d'une composition contenant dans un milieu approprié pour la teinture, au moins un composé choisi parmi un aldehyde, une cétone, une quinone et un dérivé de la di-imino-isoindoline ou de la 3-amino-isoindolone de façon à permettre le développement d'une teinture avec lesdites fibres kératiniques.

8. Procédé selon la revendication 7, **caractérisé par le fait qu'**il consiste à mélanger les composants (A) et (B) juste avant emploi, à appliquer immédiatement la composition résultante sur les fibres kératiniques et à laisser agir pendant 1 à 60 minutes et préférentiellement pendant 1 à 30 minutes ; les fibres kératiniques étant ensuite rincées, lavées au shampooing, rincées à nouveau, puis séchées.

9. Procédé selon la revendication 7 ou 8, **caractérisé par le fait qu'**il consiste à appliquer sur les fibres kératiniques le composant (A), suivie ou précédée de l'application sur lesdites fibres du composant (B), à laisser agir chaque composant pendant 1 à 60 minutes et préférentiellement pendant 1 à 30 minutes, à procéder éventuellement au rinçage à l'eau entre chaque application; les fibres kératiniques étant ensuite rincées, lavées au shampooing, rincées à nouveau, puis séchées.

10. Agent de teinture des fibres kératiniques, et en particulier des fibres kératiniques humaines telles que les cheveux, **caractérisé par le fait qu'**il comporte les composants (A) et (B) tels que définis dans la revendication 7, sous forme séparée; les composants (A) et (B) étant destinés à être, soit mélangés tout juste avant emploi, soit appliqués de façon successive sur les fibres à traiter.

11. Dispositif à plusieurs compartiments ou "kit de teinture", **caractérisé par le fait qu'**il comporte au moins deux compartiments dont un renferme le composant (A) tel que défini dans la revendication 7, et le second renferme le composant (B) tel que défini dans la revendication 7.

12. Dispositif selon la revendication 11, **caractérisé par le fait que** le composant (A) et/ou le composant (B) se présente(nt) sous forme de composition anhydre et qu'il comporte un troisième compartiment contenant un milieu aqueux cosmétiquement acceptable approprié pour la teinture destiné à être mélangé avant emploi dans l'un ou les deux premiers compartiments renfermant chaque composant (A) ou (B).

## Patentansprüche

1. Verwendung mindestens einer Verbindung mit aktivem Methylen, die eine Methylengruppe aufweist, welche mit zwei Gruppen mit -I-Effekt oder -M-Effekt substituiert ist, und die unter den folgenden Verbindungen ausgewählt ist, zum Färben von Keratinfasern:
1) Verbindungen der folgenden Formel (I): 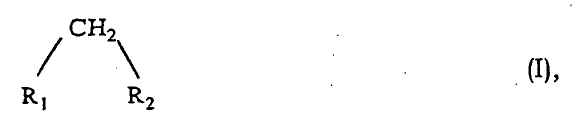 worin bedeuten:
R₁ eine Gruppe -COR oder -COOR, wobei R ein Wasserstoffatom oder eine Alkylgruppe bedeutet,
R₂ die für R₁ angegebenen Gruppen, eine Nitrilgruppe, eine unsubstituierte Arylgruppe oder eine Arylgruppe, die substituiert ist mit einem Halogen, C₁₋₄-Alkyl, C₁₋₄-Alkoxy, Nitro, Hydroxy, Carboxy, C₁₋₄-Acetyloxy, Carboxamid, Sulfonamid, einer Sulfonsäuregruppe, Nitril, -CF₃, -OCF₃ oder mit einer Cycloalkyl- oder Arylgruppe, die mit C₁₋₄-Alkyl substituiert sein kann;
eine Alkylarylgruppe, wobei die Alkylgruppe unsubstituiert sein kann oder mit einem Halogenatom, einer Cyanogruppe oder einer Hydroxygruppe substituiert ist und die Arylgruppe unsubstituiert vorliegt oder substituiert ist mit einem Halogen, C₁₋₄-Alkyl, C₁₋₄-Alkoxy, Nitro, Hydroxy, Carboxy, C₁₋₄-Acetyloxy, Carboxamid, Sulfonamid, einer Sulfonsäuregruppe, Nitril, -CF₃, -OCF₃ oder einer Cycloalkyl- oder Arylgruppe, die mit einer C₁₋₄-Alkylgruppe substituiert sein kann; oder ein Heterocyclus, der unsubstituiert vorliegt oder substituiert ist mit einem Halogen, C₁₋₄-Alkyl, C₁₋₄-Alkoxy, Nitro, Hydroxy, Carboxy, C₁₋₄-Acetyloxy, Carboxamid, Sulfonamid, einer Sulfonsäuregruppe, Nitril, -CF₃, -OCF₃ oder einer Cycloalkyl- oder Arylgruppe, die mit einer C₁₋₄-Alkylgruppe substituiert sein kann;
2) Verbindungen der folgenden Formel (II): 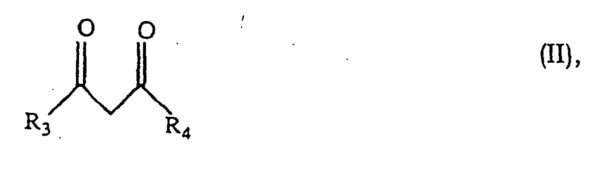 worin bedeuten:
R₃ die für R₂ angegebenen Gruppen,
R₄ eine Alkylgruppe, die unsubstituiert vorliegt oder mit einem Halogenatom, einer Cyanogruppe oder einer Hydroxygruppe substituiert ist, wobei sie die Gruppen Trifluormethyl, δ-Chlorpropyl, β-Cyanoethyl oder β-Hydroxyethyl bedeuten kann; Acetyloxy; Cycloalkyl;
eine Alkylarylgruppe, wobei die Alkylgruppe unsubstituiert vorliegt oder mit einem Halogenatom, einer Cyanogruppe oder einer Hydroxygruppe substituiert ist und die Arylgruppe unsubstituiert vorliegt oder substituiert ist mit einem Halogen, C₁₋₄-Alkyl, C₁₋₄-Alkoxy, Nitro, Hydroxy, Carboxy, C₁₋₄-Acetyloxy, Carboxamid, Sulfonamid, einer Sulfonsäuregruppe, Nitril, -CF₃, -OCF₃ oder mit einer Cycloalkyl- oder Arylgruppe, die mit einer C₁₋₄-Alkylgruppe substituiert sein kann;
eine Aralkylgruppe; eine Arylgruppe, die unsubstituiert vorliegt oder substituiert ist mit einem Halogen, C₁₋₄-Alkyl, C₁₋₄-Alkoxy, Nitro, Hydroxy, Carboxy, C₁₋₄-Acetyloxy, Carboxamid, Sulfonamid, einer Sulfonsäuregruppe, Nitril, -CF₃, -OCF₃ oder mit einer Cycloalkyl- oder Arylgruppe, die mit einer C₁₋₄-Alkylgruppe substituiert sein kann;
eine Aminoarylgruppe, die unsubstituiert vorliegt oder substituiert ist mit einem Halogen, C₁₋₄-Alkyl, C₁₋₄-Alkoxy, Nitro, Hydroxy, Carboxy, C₁₋₄-Acetyloxy, Carboxamid, Sulfonamid, einer Sulfonsäuregruppe, Nitril, -CF₃, -OCF₃ oder mit einer Cycloalkyl- oder Arylgruppe, die mit einer C₁₋₄-Alkylgruppe substituiert sein kann; und
einen Heterocyclus, der unsubstituiert vorliegt oder substituiert ist mit einem Halogen, C₁₋₄-Alkyl, C₁₋₄-Alkoxy, Nitro, Hydroxy, Carboxy, C₁₋₄-Acetyloxy, Carboxamid, Sulfonamid, einer Sulfonsäuregruppe, Nitril, -CF₃, -OCF₃ oder mit einer Cycloalkyl- oder Arylgruppe, die mit einer C₁₋₄-Alkylgruppe substituiert sein kann;
3) Verbindungen der folgenden Formel (III): 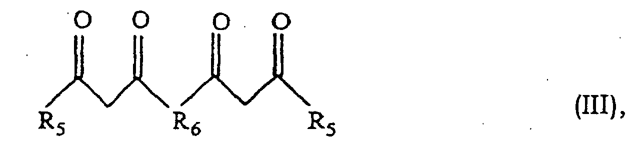 worin bedeuten:
R₅ die für die Gruppe R₂ angegebenen Bedeutungen,
R₆ eine Arylgruppe, die unsubstituiert vorliegt oder substituiert ist mit einem Halogen, C₁₋₄-Alkyl, C₁₋₄-Alkoxy, Nitro, Hydroxy, Carboxy, C₁₋₄-Acetyloxy, Carboxamid, Sulfonamid, einer Sulfonsäuregruppe, Nitril, -CF₃, -OCF₃ oder mit einer Cycloalkyl- oder Arylgruppe, die mit einer C₁₋₄-Alkylgruppe substituiert sein kann;
eine Aralkylgruppe, wobei die Alkylgruppe unsubstituiert ist oder mit einem Halogenatom, einer Cyanogruppe oder einer Hydroxygruppe substituiert ist und die Arylgruppe unsubstituiert vorliegt oder substituiert ist mit einem Halogen, C₁₋₄-Alkyl, C₁₋₄-Alkoxy, Nitro, Hydroxy, Carboxy, C₁₋₄-Acetyloxy, Carboxamid, Sulfonamid, einer Sulfonsäuregruppe, Nitril, -CF₃, -OCF₃ oder mit einer Cycloalkyl- oder Arylgruppe, die mit einer C₁₋₄-Alkylgruppe substituiert sein kann;
eine Aminoarylgruppe, die unsubstituiert vorliegt oder substituiert ist mit einem Halogen, C₁₋₄-Alkyl, C₁₋₄-Alkoxy, Nitro, Hydroxy, Carboxy, C₁₋₄-Acetyloxy, Carboxamid, Sulfonamid, einer Sulfonsäuregruppe, Nitril, -CF₃, -OCF₃ oder mit einer Cycloalkyl- oder Arylgruppe, die mit einer C₁₋₄-Alkylgruppe substituiert sein kann; oder
einen Heterocyclus, der unsubstituiert vorliegt oder substituiert ist mit einem Halogen, C₁₋₄-Alkyl, C₁₋₄-Alkoxy, Nitro, Hydroxy, Carboxy, C₁₋₄-Acetyloxy, Carboxamid, Sulfonamid, einer Sulfonsäuregruppe, Nitril, -CF₃, -OCF₃ oder mit einer Cycloalkyl- oder Arylgruppe, die mit einer C₁₋₄-Alkylgruppe substituiert sein kann;
4) Pyrazolderivaten (i) der folgenden Formeln (IV) und (V): 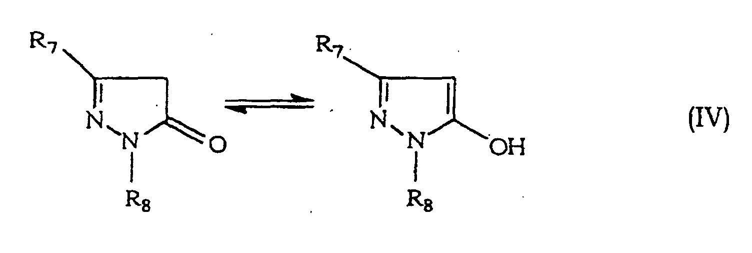 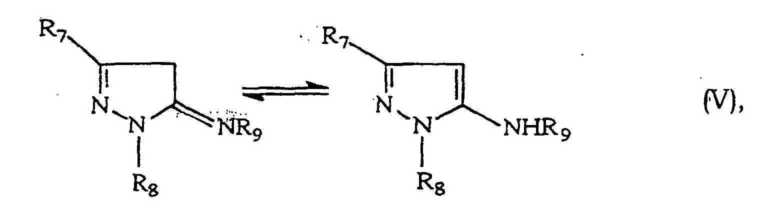 worin bedeuten:
R₇ und R₈, die gleich oder verschieden sind, die für R₄ angegebenen Gruppen,
R₉ ein Wasserstoffatom oder eine Alkylgruppe, die unsubstituiert vorliegt oder substituiert ist mit einem Halogenatom, einer Cyanogruppe oder einer Hydroxygruppe, wobei sie die Gruppen Trifluormethyl, δ-Chlorpropyl, β-Cyanoethyl oder β-Hydroxyethyl bedeuten kann;
und (ii) aus zwei Pyrazolringen der Formel (IV) oder (V) gebildeten Derivaten, die über R₇ oder R₈ verbunden sind;
5) Barbitursäurederivaten (i) der folgenden Formel (VI): 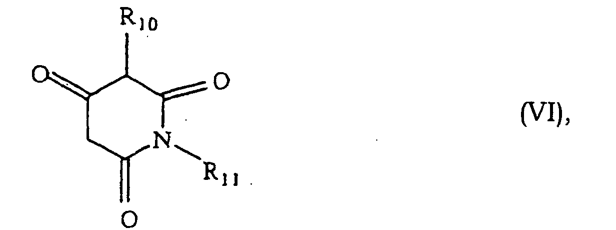 worin bedeuten:
R₁₀ und R₁₁, die gleich oder verschieden sind, eine Alkylgruppe, die unsubstituiert ist oder mit einem Halogenatom, einer Cyanogruppe oder einer Hydroxygruppe substituiert ist, wobei sie die Gruppen Trifluormethyl, δ-Chlorpropyl, β-Cyanoethyl oder β-Hydroxyethyl bedeuten kann; eine Alkenylgruppe; eine Cycloalkylgruppe; eine Alkylarylgruppe, wobei die Alkylgruppe unsubstituiert vorliegt oder substituiert ist mit einem Halogenatom, eine Cyanogruppe oder einer Hydroxygruppe und wobei die Arylgruppe unsubstituiert vorliegt oder substituiert ist mit einem Halogen, C₁₋₄-Alkyl, C₁₋₄-Alkoxy, Nitro, Hydroxy, Carboxy, C₁₋₄-Acetyloxy, Carboxamid, Sulfonamid, einer Sulfonsäuregruppe, Nitril, -CF₃, -OCF₃ oder mit einer Cycloalkyl- oder Arylgruppe, die mit einer C₁₋₄-Alkylgruppe substituiert sein kann;
oder eine Arylgruppe, die unsubstituiert vorliegt oder substituiert ist mit einem Halogen, C₁₋₄-Alkyl, C₁₋₄-Alkoxy, Nitro, Hydroxy, Carboxy, C₁₋₄-Acetyloxy, Carboxamid, Sulfonamid, einer Sulfonsäuregruppe, Nitril, -CF₃, -OCF₃ oder mit einer Cycloalkyl- oder Arylgruppe, die mit einer C₁₋₄-Alkylgruppe substituiert sein kann;
und (ii) aus zwei Ringen der Formel (VI) gebildeten Derivaten, die über R₁₀ oder R₁₁ verbunden sind;
6) Pyridinderivaten der Formel (VII): 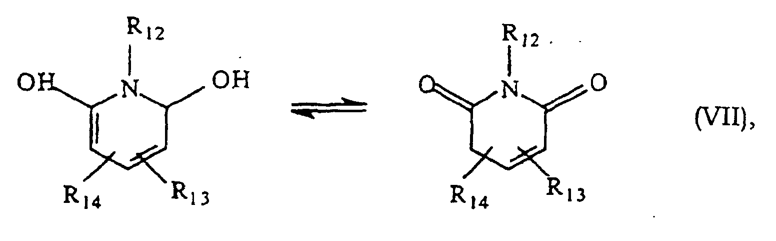 worin bedeuten:
R₁₂ eine Alkylgruppe, die unsubstituiert vorliegt oder substituiert ist mit einem Halogenatom, einer Cyanogruppe oder einer Hydroxygruppe, wobei sie die Gruppen Trifluormethyl, δ-Chlorpropyl, β-Cyanoethyl oder β-Hydroxyethyl bedeuten kann; eine unsubstituierte Arylgruppe oder eine Arylgruppe, die substituiert ist mit einem Halogen, C₁₋₄-Alkyl, C₁₋₄-Alkoxy, Nitro, Hydroxy, Carboxy, C₁₋₄-Acetyloxy, Carboxamid, Sulfonamid, einer Sulfonsäuregruppe, Nitril, -CF₃, -OCF₃ oder mit einer Cycloalkyl- oder Arylgruppe, die mit einer C₁₋₄-Alkylgruppe substituiert sein kann;
R₁₃ ein Wasserstoffatom, eine Alkylgruppe, die unsubstituiert ist oder mit einem Halogenatom, einer Cyanogruppe oder einer Hydroxygruppe substituiert ist, wobei sie die Gruppen Trifluormethyl, δ-Chlorpropyl, β-Cyanoethyl oder β-Hydroxyethyl bedeuten kann; eine Arylgruppe, die unsubstituiert vorliegt oder substituiert ist mit einem Halogen, C₁₋₄-Alkyl, C₁₋₄-Alkoxy, Nitro, Hydroxy, Carboxy, C₁₋₄-Acetyloxy, Carboxamid, Sulfonamid, einer Sulfonsäuregruppe, Nitril, -CF₃, -OCF₃ oder mit einer Cycloalkyl- oder Arylgruppe, die mit einer C₁₋₄-Alkylgruppe substituiert sein kann;
R₁₄ ein Wasserstoffatom, eine Nitrilgruppe, eine Alkylgruppe, die unsubstituiert ist oder mit einem Halogenatom, einer Cyanogruppe oder einer Hydroxygruppe substituiert ist, wobei sie die Gruppen Trifluormethyl, δ-Chlorpropyl, β-Cyanoethyl oder β-Hydroxyethyl bedeuten kann, eine Gruppe -COOR, wobei R ein Wasserstoffatom oder eine Alkylgruppe bedeutet, die unsubstituiert vorliegt oder substituiert ist mit einem Halogenatom, einer Cyanogruppe oder einer Hydroxygruppe, wobei sie die Gruppen Trifluormethyl, δ-Chlorpropyl, β-Cyanoethyl oder β-Hydroxyethyl bedeuten kann;
7) Derivaten der folgenden Formel (VIII): 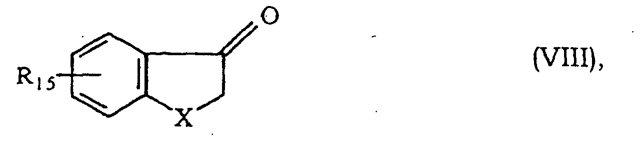 worin bedeuten:
X ein Sauerstoffatom, ein Schwefelatom, ein Stickstoffatom oder eine Gruppe NR', wobei R' eine Alkylgruppe bedeutet,
R₁₅ ein Wasserstoffatom, ein Chloratom, ein Bromatom, eine Hydroxygruppe, eine Nitrogruppe, eine Alkylgruppe, eine Alkoxygruppe, eine Carboxamidgruppe, eine Sulfonamidgruppe oder eine Nitrilgruppe;
8) Derivaten der folgenden Formeln (IX) und (X): 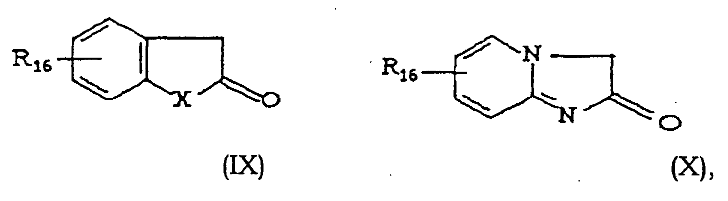 worin bedeuten:
X ein Sauerstoffatom, ein Schwefelatom, ein Stickstoffatom oder eine Gruppe NR', wobei R' eine Alkylgruppe bedeutet,
R₁₆ die Atome und Gruppen, die für R₁₅ angegeben wurden;
9) Derivaten der folgenden Formel (XI): 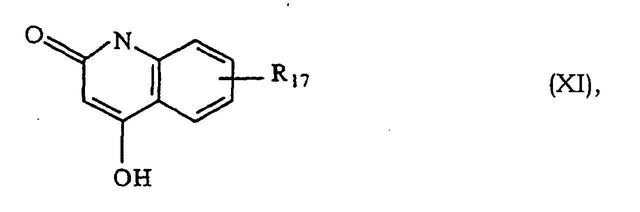 worin bedeuten:
R₁₇ ein Wasserstoffatom, eine Hydroxygruppe oder eine Alkylgruppe, die unsubstituiert ist oder mit einem Halogenatom, einer Cyanogruppe oder einer Hydroxygruppe substituiert ist, wobei sie die Gruppen Trifluormethyl, δ-Chlorpropyl, β-Cyanoethyl oder β-Hydroxyethyl bedeuten kann; oder eine Arylgruppe, die unsubstituiert ist oder substituiert ist mit einem Halogen, C₁₋₄-Alkyl, C₁₋₄-Alkoxy, Nitro, Hydroxy, Carboxy, C₁₋₄-Acetyloxy, Carboxamid, Sulfonamid, einer Sulfonsäuregruppe, Nitril, -CF₃, -OCF₃ oder mit einer Cycloalkyl- oder Arylgruppe, die mit einer C₁₋₄-Alkylgruppe substituiert sein kann; oder
eine Alkylarylgruppe, wobei die Alkylgruppe unsubstituiert ist oder mit einem Halogenatom, einer Cyanogruppe oder einer Hydroxygruppe substituiert ist und die Arylgruppe unsubstituiert vorliegt oder substituiert ist mit einem Halogen, C₁₋₄-Alkyl, C₁₋₄-Alkoxy, Nitro, Hydroxy, Carboxy, C₁₋₄-Acetyloxy, Carboxamid, Sulfonamid, einer Sulfonsäuregruppe, Nitril, -CF₃, -OCF₃ oder mit einer Cycloalkyl- oder Arylgruppe, die mit einer C₁₋₄-Alkylgruppe substituiert sein kann;
10) Indandionderivaten der folgenden Formel (XII): 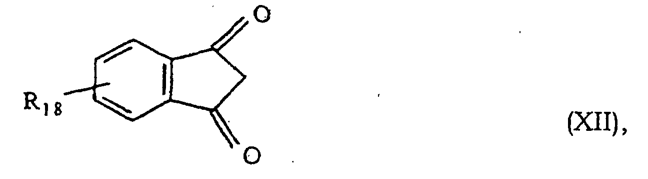 worin bedeuten:
R₁₈ Wasserstoff, Chlor, Brom, Nitro, Alkyl, Alkoxy, Carboxamid, Sulfonamid oder Nitril;
11) Derivaten der folgenden Formel (XIII): 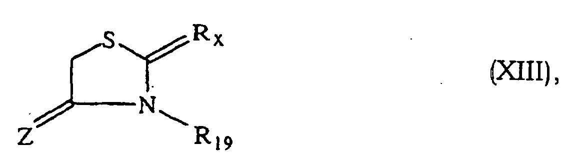 worin bedeuten:
Z O oder NR mit R = H oder Alkyl;
Rₓ ein Schwefelatom oder NR, wobei R ein Wasserstoffatom oder eine Alkylgruppe bedeutet;
R₁₉ ein Wasserstoffatom, Alkyl, Alkoxy, Nitro oder Nitril;
12) Dioxopyrazolderivaten der folgenden Formel (XIV): 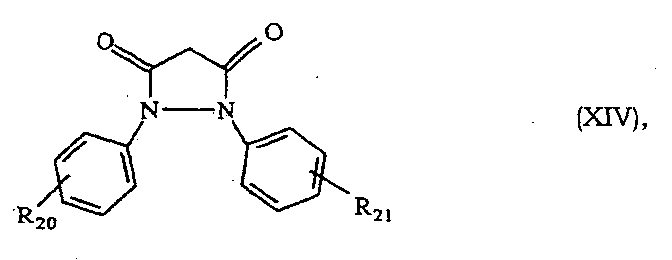 worin bedeuten;
R₂₀ und R₂₁, die gleich oder verschieden sind, ein Wasserstoffatom, Alkyl, Alkoxy, Nitro oder Nitril;
13) 5-Oxoimidazolderivaten der folgenden Formel (XV): 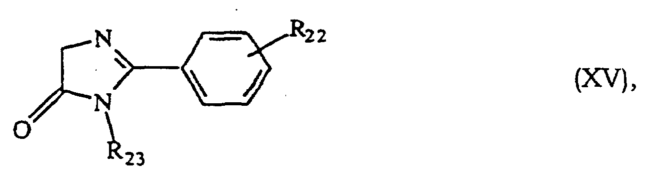 worin bedeuten:
R₂₂ ein Wasserstoffatom oder Alkyl,
R₂₃ ein Wasserstoffatom, Alkyl, Alkoxy, Nitro oder Nitril;
14) Dehydrobutyrolactonderivaten der folgenden Formel (XVI): 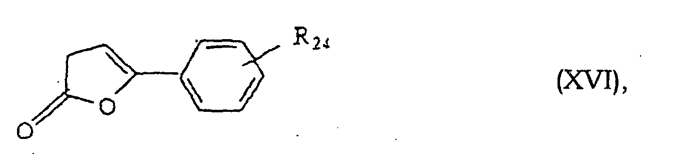 worin bedeuten:
R₂₄ ein Wasserstoffatom, Alkyl, Alkoxy, Nitro oder Nitril;
15) Verbindungen der folgenden Formel (XVII): 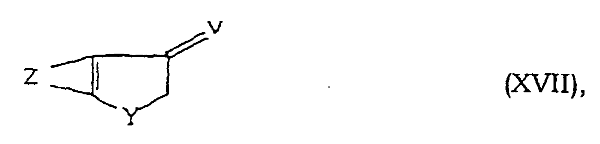 worin bedeuten:
Z einen aromatischen Ring,
V ein Sauerstoffatom oder eine Gruppe A-CH₂-E, wobei A oder E einen Substituenten, der eine Hammet-Konstante im Bereich von 0,4 bis 2,0 aufweist, oder Substituenten bedeuten, deren Summe der Hammet-Konstanten im Bereich von 0,4 bis 2,0 liegt,
Y CO, O, S, NR, wenn V von Sauerstoff verschieden ist, oder CS, C = NR₂, SO, SO₂, wobei R₁ oder R₂ Wasserstoff oder Alkyl bedeuten;
und den kosmetisch akzeptablen Salzen der oben definierten Verbindungen;
und mindestens einer Verbindung, die ausgewählt ist unter:
- Aldehyden der folgenden Formel (XVIII): 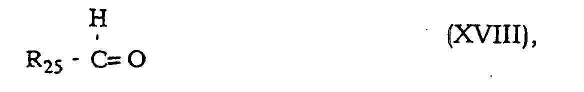 worin bedeuten:
R₂₅ eine Gruppe der folgenden Formel (XVIII A), 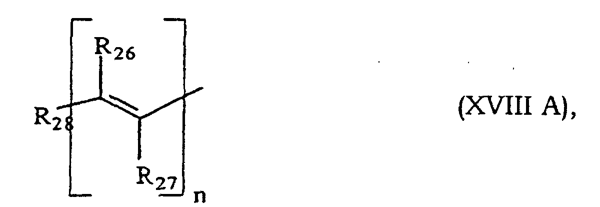 wobei R₂₆ und R₂₇, die identisch oder voneinander verschieden sind, Wasserstoff, Alkyl, Mono- oder Polyhydroxyalkyl, Alkylhydroxyalkyl, Alkoxy, -CF₃ oder -OCF₃ bedeuten,
R₂₆ und R₂₇ auch mit den Atomen, an die sie gebunden sind, einen Arylring oder einen Heterocyclus mit 5 oder 6 Bestandteilen bilden können, wobei die Ringe unsubstituiert sein können oder substituiert mit einem Halogen, C₁₋₄-Alkyl, C₁₋₄-Alkoxy, Nitro, Hydroxy, Carboxy, C₁₋₄-Acetyloxy, Carboxamid, Sulfonamid, einer Sulfonsäuregruppe, Nitril, -CF₃, -OCF₃ oder mit einer Cycloalkyloder Arylgruppe, die mit einer C₁₋₄-Alkylgruppe substituiert sein kann;
n 0 oder eine ganze Zahl von 1 bis 3 bedeutet,
R₂₈ bedeutet die für R₂₆ angegebenen Substituenten, eine Arylgruppe, eine Alkylarylgruppe, wobei die Alkylgruppe unsubstituiert vorliegt oder substituiert ist mit einem Halogen, einer Cyanogruppe oder einer Hydroxygruppe und die Arylgruppe unsubstituiert vorliegt oder substituiert ist mit einem Halogen, C₁₋₄-Alkyl, C₁₋₄-Alkoxy, Nitro, Hydroxy, Carboxy, C₁₋₄-Acetyloxy, Carboxamid, Sulfonamid, einer Sulfonsäuregruppe, Nitril, -CF₃, -OCF₃ oder mit einer Cycloalkyl- oder Arylgruppe, die mit einer C₁₋₄-Alkylgruppe substituiert sein kann;
einen 5- oder 6-gliedrigen Heterocyclus, der unsubstituiert vorliegt oder substituiert ist mit einem Halogen, C₁₋₄-Alkyl, C₁₋₄-Alkoxy, Nitro, Hydroxy, Carboxy, C₁₋₄-Acetyloxy, Carboxamid, Sulfonamid, einer Sulfonsäuregruppe, Nitril, -CF₃, -OCF₃ oder mit einer Cycloalkyl- oder Arylgruppe, die mit einer C₁₋₄-Alkylgruppe substituiert sein kann; oder den kosmetisch akzeptablen Salzen dieser Verbindungen;
- Ketonen der folgenden Formeln (XIX) oder (XX): 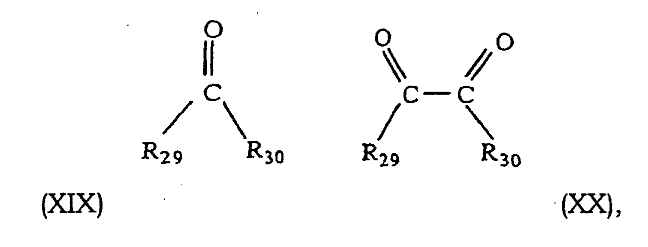 worin bedeuten:
R₂₉ die für R₂₅ angegebenen Substituenten,
R₃₀ eine Alkylgruppe, eine Mono- oder Polyhydroxyalkylgruppe, eine Alkylhydroxyalkylgruppe; eine Arylgruppe, eine Alkylarylgruppe, einen 5- oder 6-gliedrigen Heterocyclus, der unsubstituiert vorliegt oder substituiert ist mit einem Halogen, C₁₋₄-Alkyl, C₁₋₄-Alkoxy, Nitro, Hydroxy, Carboxy, C₁₋₄-Acetyloxy, Carboxamid, Sulfonamid, einer Sulfonsäuregruppe, Nitril, -CF₃, -OCF₃ oder mit einer Cycloalkyl- oder Arylgruppe, die mit einer C₁₋₄-Alkylgruppe substituiert sein kann;
wobei R₂₉ und R₃₀ auch mit den Atomen, an die sie gebunden sind, einen 5- oder 6-gliedrigen Arylring oder einen Heterocyclus bilden können, der Heteroatome wie N oder S enthält, wobei der Ring oder Heterocyclus selbst an einen Arylring mit 5 oder 6 Ringbestandteilen oder einen Heterocyclus, der Heteroatome wie N oder S enthält, gebunden sein kann, wobei der Ring oder Heterocyclus unsubstituiert vorliegt oder substituiert ist mit einem Halogen, C₁₋₄-Alkyl, C₁₋₄-Alkoxy, Nitro, Hydroxy, Carboxy, C₁₋₄-Acetyloxy, Carboxamid, Sulfonamid, einer Sulfonsäuregruppe, Nitril, -CF₃, -OCF₃ oder mit einer Cycloalkyl- oder Arylgruppe, die mit einer C₁₋₄-Alkylgruppe substituiert sein kann;
oder den kosmetisch akzeptablen Salzen dieser Verbindungen;
- Chinonen der folgenden Formeln (XXI) und (XXII): 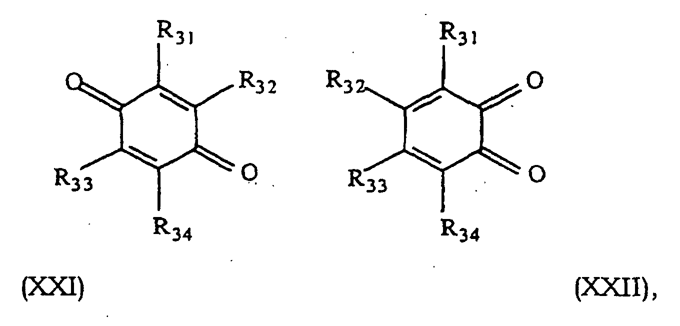 worin bedeuten;
R₃₁ ein Wasserstoffatom, ein Halogenatom, eine Sulfonsäuregruppe oder eine Alkoxygruppe;
R₃₂, R₃₃ und R₃₄, die gleich oder verschieden sind, ein Wasserstoffatom, ein Halogenatom, Hydroxy, Alkyl, Monooder Polyhydroxyalkyl, Alkylhydroxyalkyl, Alkylsulfonyl, Carboxyalkyl, Aminoalkyl, Alkylaminoalkyl, (Dihydroxy)-alkylaminoalkyl oder Alkyl-NR'R", wobei R' und R" Alkyl bedeuten oder gemeinsam mit dem Stickstoffatom, an das sie gebunden sind, einen Arylring oder einen Heterocyclus mit 5 oder 6 Ringbestandteilen bilden können, Aryl, eine Aminogruppe, die mit Alkyl oder Hydroxyalkyl substituiert sein kann, wobei R₃₁ und R₃₂, R₃₁ und R₃₃ oder R₃₃ und
R₃₄ gemeinsam mit den Atomen, an die sie gebunden sind, einen Arylring oder einen Heterocyclus mit 5 oder 6 Ringbestandteilen bilden können, der unsubstituiert vorliegt oder substituiert ist mit einem Halogen, C₁₋₄-Alkyl, C₁₋₄-Alkoxy, Nitro, Hydroxy, Carboxy, C₁₋₄-Acetyloxy, Carboxamid, Sulfonamid, einer Sulfonsäuregruppe, Nitril, -CF₃, -OCF₃ oder mit einer Cycloalkyl- oder Arylgruppe, die mit einer C₁₋₄-Alkylgruppe substituiert sein kann; oder den kosmetisch akzeptablen Salzen dieser Verbindungen,
- und Diiminoisoindolinderivaten oder 3-Aminoisoindolonderivaten der folgenden Formel (XXIII): 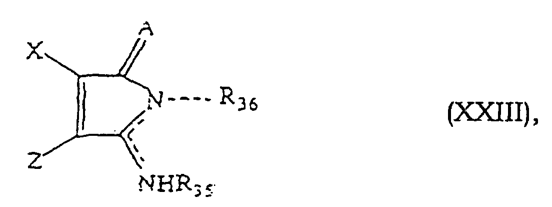 worin bedeuten:
R₃₅ und R₃₆, die gleich oder verschieden sind, ein Wasserstoffatom, Alkyl, Mono- oder Polyhydroxyalkyl, Alkylhydroxyalkyl, Aminoalkyl, Alkylaminoalkyl, (Dihydroxy)-alkylaminoalkyl oder eine Gruppe Alkyl-NR'R", wobei R' und R" Alkyl bedeuten oder gemeinsam mit dem Stickstoffatom, an das sie gebunden sind, einen Arylring oder einen Heterocyclus mit 5 oder 6 Ringbestandteilen bilden können, A ein Sauerstoffatom oder NH, wobei X und Z gemeinsam einen Arylring oder einen Heterocyclus mit 5 oder 6 Ringbestandteilen bilden, der unsubstituiert ist oder substituiert mit einem Halogen, C₁₋₄-Alkyl, C₁₋₄-Alkoxy, Nitro, Hydroxy, Carboxy, C₁₋₄-Acetyloxy, Carboxamid, Sulfonamid, einer Sulfonsäuregruppe, Nitril, -CF₃, -OCF₃ oder mit einer Cycloalkyl- oder Arylgruppe, die mit einer C₁₋₄-Alkylgruppe substituiert sein kann;
oder den kosmetisch akzeptablen Salzen dieser Verbindungen,
wobei mit diesen Verbindungen durch eine Reaktion ohne Oxidationsmittel die Keratinfasern gefärbt werden können;
sowie den Kombinationen der folgenden Verbindungen:
- 3-Imino-3H-isoindol-1-ylamin und Benzoylacetonitril;
- 4-Dimethylaminobenzaldehyd und Benzoylacetonitril;
- 1,4-Naphthochinon und Benzoylacetonitril;
- Isatin und Benzoylacetonitril;
- 3-Imino-3H-isoindol-1-ylamin und 5-Amino-2H-pyrazol-3-ol;
- 4-Dimethylaminobenzaldehyd und 5-Amino-2H-pyrazol-3-ol;
- 1,4-Naphthochinon und 5-Amino-2H-pyrazol-3-ol;
- 3-Imino-3H-isoindol-1-ylamin und 3-Amino-1-phenyl-2-pyrazolin-5-on;
- 4-Dimethylaminobenzaldehyd und 3-Amino-1-phenyl-2-pyrazolin-5-on;
- 1,4-Naphthochinon und 3-Amino-1-phenyl-2-pyrazolin-5-on.

2. Zusammensetzung zum Färben von Keratinfasern, insbesondere menschlichen Keratinfasern, beispielsweise zum Färben der Haare, **dadurch gekennzeichnet, dass** sie mindestens eine Verbindung mit aktivem Methylen, die unter den in Anspruch 1 definierten Verbindungen ausgewählt ist, und mindestens eine Verbindung, die unter Aldehyden, Ketonen, Chinonen und Diiminoisoindolinderivaten oder 3-Aminoisoindolonderivaten nach Anspruch 1 ausgewählt ist, in einem zum Färben geeigneten Medium enthält.

3. Zusammensetzung zum Färben nach Anspruch 2, **dadurch gekennzeichnet, dass** sie einen pH-Wert im Bereich von 2 bis 11 aufweist.

4. Zusammensetzung nach Anspruch 2 oder 3, **dadurch gekennzeichnet, dass** die Verbindung mit aktivem Methylen in einer Konzentration von 0,01 bis 10 % und vorzugsweise 0,05 bis 5 Gew.-%, bezogen auf das Gesamtgewicht der Zusammensetzung, enthalten ist.

5. Zusammensetzung nach einem der Ansprüche 2 bis 4, **dadurch gekennzeichnet, dass** die Verbindung, die unter Aldehyden, Ketonen, Chinonen und Diiminoisoindolinderivaten oder 3-Aminoisoindolonderivaten ausgewählt ist, in einer Konzentration von 0,01 bis 10 % und vorzugsweise 0,05 bis 5 Gew.-%, bezogen auf das Gesamtgewicht der Zusammensetzung, enthalten ist.

6. Zusammensetzung nach einem der Ansprüche 2 bis 5, **dadurch gekennzeichnet, dass** das zum Färben geeignete Medium ein wässriges Medium ist, das aus Wasser und/oder organischen Lösungsmitteln, die unter Alkoholen, Glykolen und Glykolethern ausgewählt sind, in Mengenanteilen von 0,5 bis 20 Gew.-%, bezogen auf das Gesamtgewicht der Zusammensetzung, besteht.

7. Verfahren zum Färben von Keratinfasern, insbesondere menschlichen Keratinfasern, wie Haaren, **dadurch gekennzeichnet, dass** es darin besteht, auf die Fasern eine Komponente (A), die aus einer Zusammensetzung besteht, die in einem zum Färben geeigneten Medium mindestens eine in Anspruch 1 definierte Verbindung mit aktivem Methylen enthält, und mindestens eine Komponente (B) aufzutragen, die aus einer Zusammensetzung besteht, die in einem zum Färben geeigneten Medium mindestens eine Verbindung enthält, die unter den Aldehyden, Ketonen, Chinonen, Diiminoisoindolinderivaten oder 3-Aminoisoindolonderivaten ausgewählt ist, um die Keratinfasern zu färben.

8. Verfahren nach Anspruch 7, **dadurch gekennzeichnet, dass** es darin besteht, die Komponenten (A) und (B) kurz vor der Anwendung zu vermischen, die resultierende Zusammensetzung sofort auf die Keratinfasern aufzutragen und 1 bis 60 min und vorzugsweise 1 bis 30 min einwirken zu lassen, wobei die Keratinfasern anschließend gespült, mit Haarwaschmittel gewaschen, nochmals gespült und dann getrocknet werden.

9. Verfahren nach Anspruch 7 oder 8, **dadurch gekennzeichnet, dass** es darin besteht, auf die Keratinfasern die Komponente (A) aufzutragen, wobei die Komponente (B) zuvor oder danach aufgetragen wird, jede Komponente 1 bis 60 min und vorzugsweise 1 bis 30 min einwirken zu lassen und zwischen den Anwendungen gegebenenfalls mit Wasser zu spülen, wobei die Keratinfasern dann gespült, mit Haarwaschmittel gewaschen, nochmals gespült und dann getrocknet werden.

10. Mittel zum Färben von Keratinfasern und insbesondere menschlichen Keratinfasern, wie zum Färben der Haare, **dadurch gekennzeichnet, dass** es die in Anspruch 7 definierten Komponenten (A) und (B) in getrennter Form umfasst, wobei die Komponenten (A) und (B) entweder kurz vor der Anwendung vermischt oder nacheinander auf die zu behandelnden Fasern aufgetragen werden sollen.

11. Vorrichtung mit mehreren Abteilungen oder Kit zum Färben, **dadurch gekennzeichnet, dass** sie mindestens zwei Abteilungen aufweist, wobei eine Abteilung die in Anspruch 7 definierte Komponente (A) und eine zweite Abteilung die in Anspruch definierte Komponente (B) umfasst.

12. Vorrichtung nach Anspruch 11, **dadurch gekennzeichnet, dass** die Komponente (A) und/oder die Komponente (B) in Form einer wasserfreien Zusammensetzung vorliegt und die Vorrichtung eine dritte Abteilung aufweist, die ein zum Färben geeignetes, kosmetisch akzeptables Medium enthält, das vor der Anwendung in einer oder beiden Abteilungen vermischt werden soll, die die Komponente (A) oder (B) enthalten.

## Claims

1. Use, for dyeing keratin fibres, of at least one compound containing active methylene comprising a methylene group substituted with two groups with an electron-withdrawing or mesomeric effect chosen from:
1) the compounds of formula (I) below: 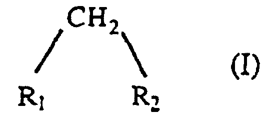 in which:
R₁ denotes a group -COR or -COOR with R denoting a hydrogen atom or an alkyl group,
R₂ denotes the groups denoted by R₁, a nitrile group, an aryl group unsubstituted or substituted with a halogen, with a C₁-C₄ alkyl, C₁-C₄ alkoxy, a nitro group, a hydroxyl group, a carboxyl group, a C₁-C₄ acyloxy group, a carboxamide group, a sulphonamide, sulpho, nitrile, -CF₃ or -OCF₃ group, or with a cycloalkyl or aryl radical which may be substituted with a C₁-C₄ alkyl;
an alkylaryl group, the alkyl being unsubstituted or substituted with a halogen atom, cyano or hydroxyl radical and the aryl being unsubstituted or substituted with a halogen, with a C₁-C₄ alkyl, a C₁-C₄ alkoxy, a nitro group, a hydroxyl group, a carboxyl group, a C₁-C₄ acyloxy group, a carboxamide group, a sulphonamide, sulpho, nitrile, -CF₃ or -OCF₃ group, or with a cycloalkyl or aryl radical which may be substituted with a C₁-C₄ alkyl;
or a heterocycle which is unsubstituted or substituted with a halogen, with a C₁-C₄ alkyl, a C₁-C₄ alkoxy, a nitro group, a hydroxyl group, a carboxyl group, a C₁-C₄ acyloxy group, a carboxamide group, a sulphonamide, sulpho, nitrile, -CF₃ or -OCF₃ group, or with a cycloalkyl or aryl radical which may be substituted with a C₁-C₄ alkyl;
2) the compounds of formula (II) below: 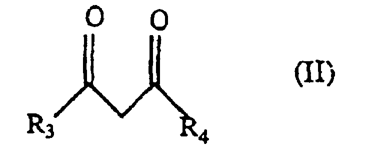 in which:
R₃ denotes the groups denoted by R₂
R₄ denotes an alkyl group unsubstituted or substituted with a halogen atom, a cyano or hydroxyl radical, which may represent the trifluoromethyl, δ-chloropropyl, β-cyanoethyl or β-hydroxyethyl radicals; an acetyloxy group; a cycloalkyl group;
an alkylaryl group, the alkyl being unsubstituted or substituted with a halogen atom, a cyano or hydroxyl radical and the aryl being unsubstituted or substituted with a halogen, with a C₁-C₄ alkyl, a C₁-C₄ alkoxy, a nitro group, a hydroxyl group, a carboxyl group, a C₁-C₄ acyloxy group, a carboxamide group, a sulphonamide, sulpho, nitrile, -CF₃ or -OCF₃ group, or with a cycloalkyl or aryl radical which may be substituted with a C₁-C₄ alkyl;
an aralkyl group; an aryl group unsubstituted or substituted with a halogen, with a C₁-C₄ alkyl, a C₁-C₄ alkoxy, a nitro group, a hydroxyl group, a carboxyl group, a C₁-C₄ acyloxy group, a carboxamide group, a sulphonamide, sulpho, nitrile, -CF₃ or -OCF₃ group, or with a cycloalkyl or aryl radical which may be substituted with a C₁-C₄ alkyl;
an aminoaryl group unsubstituted or substituted with a halogen, with a C₁-C₄ alkyl, a C₁-C₄ alkoxy, a nitro group, a hydroxyl group, a carboxyl group, a C₁-C₄ acyloxy group, a carboxamide group, a sulphonamide, sulpho, nitrile, -CF₃ or -OCF₃ group, or with a cycloalkyl or aryl radical which may be substituted with a C₁-C₄ alkyl;
or a heterocycle unsubstituted or substituted with a halogen, with a C₁-C₄ alkyl, a C₁-C₄ alkoxy, a nitro group, a hydroxyl group, a carboxyl group, a C₁-C₄ acyloxy group, a carboxamide group, a sulphonamide, sulpho, nitrile, -CF₃ or -OCF₃ group, or with a cycloalkyl or aryl radical which may be substituted with a C₁-C₄ alkyl;
3) the compounds of formula (III) below: 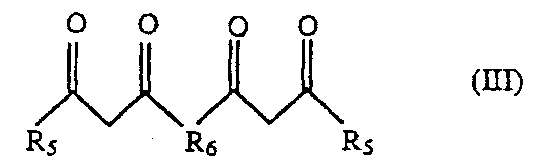 in which:
R₅ denotes the groups denoted by R₂
R₆ denotes an aryl group unsubstituted or substituted with a halogen, with a C₁-C₄ alkyl, a C₁-C₄ alkoxy, a nitro group, a hydroxyl group, a carboxyl group, a C₁-C₄ acyloxy group, a carboxamide group, a sulphonamide, sulpho, nitrile, -CF₃ or -OCF₃ group, or with a cycloalkyl or aryl radical which may be substituted with a C₁-C₄ alkyl;
an aralkyl group, the alkyl being unsubstituted or substituted with a halogen atom, a cyano or hydroxyl radical and the aryl being unsubstituted or substituted with a halogen, with a C₁-C₄ alkyl, a C₁-C₄ alkoxy, a nitro group, a hydroxyl group, a carboxyl group, a C₁-C₄ acyloxy group, a carboxamide group, a sulphonamide, sulpho, nitrile, -CF₃ or -OCF₃ group, or with a cycloalkyl or aryl radical which may be substituted with a C₁-C₄ alkyl;
an aminoaryl group unsubstituted or substituted with a halogen, with a C₁-C₄ alkyl, a C₁-C₄ alkoxy, a nitro group, a hydroxyl group, a carboxyl group, a C₁-C₄ acyloxy group, a carboxamide group, a sulphonamide, sulpho, nitrile, -CF₃ or -OCF₃ group, or with a cycloalkyl or aryl radical which may be substituted with a C₁-C₄ alkyl;
or a heterocycle unsubstituted or substituted with a halogen, with a C₁-C₄ alkyl, a C₁-C₄ alkoxy, a nitro group, a hydroxyl group, a carboxyl group, a C₁-C₄ acyloxy group, a carboxamide group, a sulphonamide, sulpho, nitrile, -CF₃ or -OCF₃ group, or with a cycloalkyl or aryl radical which may be substituted with a C₁-C₄ alkyl;
4) the pyrazole derivatives (i) of formulae (IV) and (V) below: 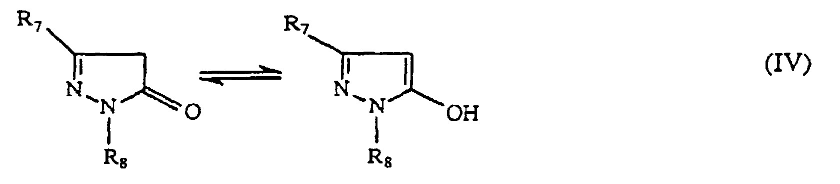 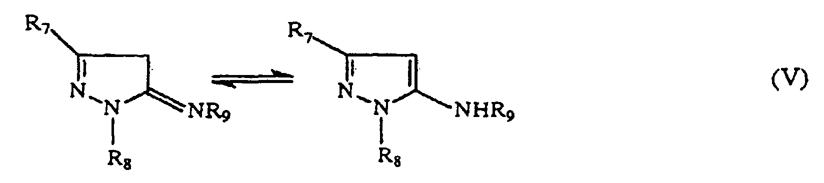 in which:
R₇ and R₈, which are identical or different, denote the groups denoted by R₄,
R₉ denotes a hydrogen atom or an alkyl group unsubstituted or substituted with a halogen atom, a cyano or hydroxyl radical, which may represent the trifluoromethyl, β-chloropropyl, β-cyanoethyl or β-hydroxyethyl radicals;
and (ii) formed by two pyrazole rings of formula (IV) or (V) linked by R₇ or R₈;
5) the barbituric acid derivatives (i) of formula (VI) below: 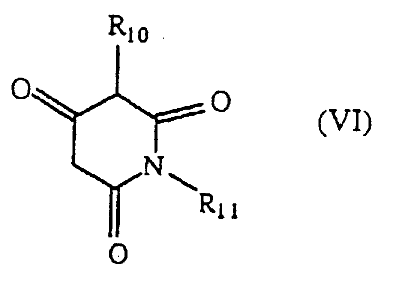 in which:
R₁₀ and R₁₁, which are identical or different, denote an alkyl group unsubstituted or substituted with a halogen atom, a cyano or hydroxyl radical, which may represent the trifluoromethyl, δ-chloropropyl, β-cyanoethyl or β-hydroxyethyl radicals; an alkenyl group; a cycloalkyl group; an alkylaryl group, the alkyl being unsubstituted or substituted with a halogen atom, a cyano or hydroxyl radical and the aryl being unsubstituted or substituted with a halogen, with a C₁-C₄ alkyl, C₁-C₄ alkoxy, a nitro group, a hydroxyl group, a carboxyl group, a C₁-C₄ acyloxy group, a carboxamide group, a sulphonamide, sulpho, nitrile, -CF₃ or -OCF₃ group, or with a cycloalkyl or aryl radical which may be substituted with a C₁-C₄ alkyl;
or an aryl group unsubstituted or substituted with a halogen, with a C₁-C₄ alkyl, C₁-C₄ alkoxy, a nitro group, a hydroxyl group, a carboxyl group, a C₁-C₄ acyloxy group, a carboxamide group, a sulphonamide, sulpho, nitrile, -CF₃ or -OCF₃ group, or with a cycloalkyl or aryl radical which may be substituted with a C₁-C₄ alkyl; and (ii) formed by two rings of formula (VI) linked by R₁₀ or R₁₁;
6) the pyridine derivatives of formula (VII): 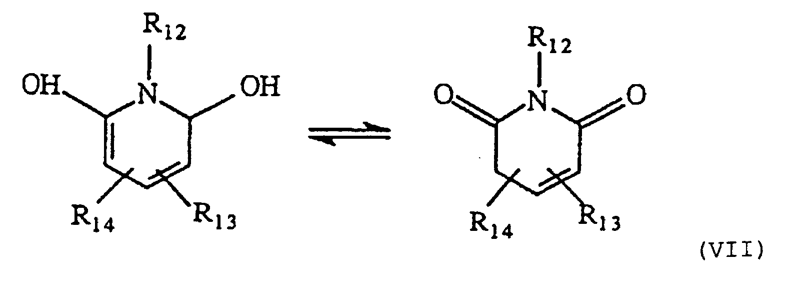 in which:
R₁₂ denotes an alkyl group unsubstituted or substituted with a halogen atom, a cyano or hydroxyl radical, which may represent the trifluoromethyl, δ-chloropropyl, β-cyanoethyl or β-hydroxyethyl radicals; an aryl group unsubstituted or substituted with a halogen, with a C₁-C₄ alkyl, a C₁-C₄ alkoxy, a nitro group, a hydroxyl group, a carboxyl group, a C₁-C₄ acyloxy group, a carboxamide group, a sulphonamide, sulpho, nitrile, -CF₃ or -OCF₃ group, or with a cycloalkyl or aryl radical which may be substituted with a C₁-C₄ alkyl;
R₁₃ denotes a hydrogen atom, an alkyl group unsubstituted or substituted with a halogen atom, a cyano or hydroxyl radical, which may represent the trifluoromethyl, δ-chloropropyl, β-cyanoethyl or β-hydroxyethyl radicals; an aryl group unsubstituted or substituted with a halogen, with a C₁-C₄ alkyl, a C₁-C₄ alkoxy, a nitro group, a hydroxyl group, a carboxyl group, a C₁-C₄ acyloxy group, a carboxamide group, a sulphonamide, sulpho, nitrile, -CF₃ or -OCF₃ group, or with a cycloalkyl or aryl radical which may be substituted with a C₁-C₄ alkyl;
R₁₄ denotes a hydrogen atom, a nitrile group, an alkyl group unsubstituted or substituted with a halogen atom, a cyano or hydroxyl radical, which may represent the trifluoromethyl, δ-chloropropyl, β-cyanoethyl or β-hydroxyethyl radicals, a group COOR, R denoting a hydrogen atom or an alkyl group unsubstituted or substituted with a halogen atom, a cyano or hydroxyl radical, which may represent the trifluoromethyl, δ-chloropropyl, β-cyanoethyl or β-hydroxyethyl radicals;
7) the derivatives of formula (VIII) below: 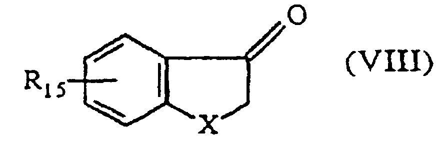 in which:
X denotes an oxygen, sulphur or nitrogen atom or a group NR', R' denoting an alkyl group,
R₁₅ denotes a hydrogen, chlorine or bromine atom or a hydroxyl, nitro, alkyl, alkoxy, carboxamide, sulphonamide or nitrile group;
8) the derivatives of formulae (IX) and (X) below: 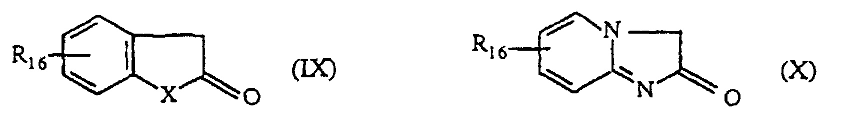 in which:
X denotes an oxygen, sulphur or nitrogen atom or a group NR', R' denoting an alkyl group,
R₁₆ denotes the atoms and groups denoted by R₁₅;
9) the derivatives of formula (XI) below: 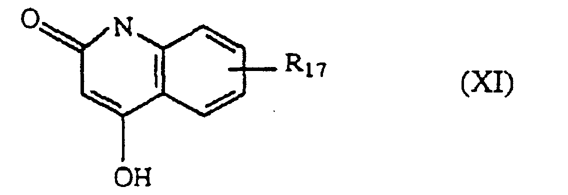 in which:
R₁₇ denotes a hydrogen atom, a hydroxyl group or an alkyl group unsubstituted or substituted with a halogen atom, a cyano or hydroxyl radical, which may represent the trifluoromethyl, δ-chloropropyl, β-cyanoethyl or β-hydroxyethyl radicals; or an aryl group unsubstituted or substituted with a halogen, with a C₁-C₄ alkyl, a C₁-C₄ alkoxy, a nitro group, a hydroxyl group, a carboxyl group, a C₁-C₄ acyloxy group, a carboxamide group, a sulphonamide, sulpho, nitrile, -CF₃ or -OCF₃ group, or with a cycloalkyl or aryl radical which may be substituted with a C₁-C₄ alkyl; or
an alkylaryl group, the alkyl being unsubstituted or substituted with a halogen atom, a cyano or hydroxyl radical and the aryl being unsubstituted or substituted with a halogen, with a C₁-C₄ alkyl, a C₁-C₄ alkoxy, a nitro group, a hydroxyl group, a carboxyl group, a C₁-C₄ acyloxy group, a carboxamide group, a sulphonamide, sulpho, nitrile, -CF₃ or -OCF₃ group, or with a cycloalkyl or aryl radical which may be substituted with a C₁-C₄ alkyl;
10) the indanedione derivatives of formula (XII) below: 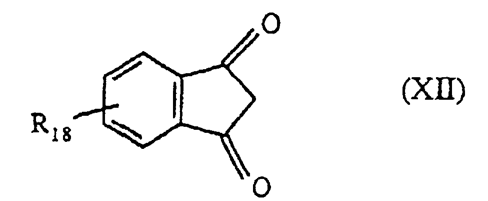 in which:
R₁₈ denotes a hydrogen, chlorine or bromine atom or a nitro, alkyl, alkoxy, carboxamide, sulphonamide or nitrile group;
11) the derivatives of formula (XIII) below: 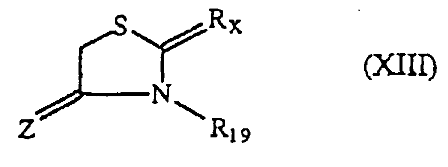 in which:
Z denotes O or NR with R = H or alkyl
Rₓ denotes a sulphur atom or NR, R denoting a hydrogen atom or an alkyl group;
R₁₉ denotes a hydrogen atom or an alkyl, alkoxy, nitro or nitrile group;
12) the dioxopyrazole derivatives of formula (XIV) below: 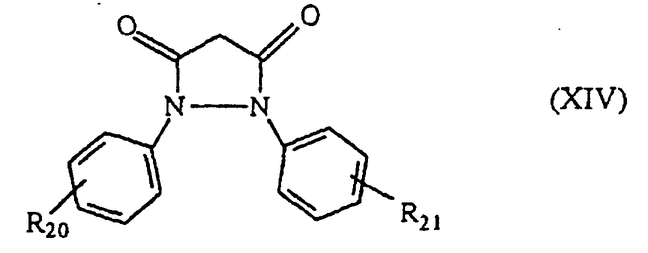 in which:
R₂₀ and R₂₁, which are identical or different, denote a hydrogen atom or an alkyl, alkoxy, nitro or nitrile group;
13) the 5-oxoimidazole derivatives of formula (XV) below: 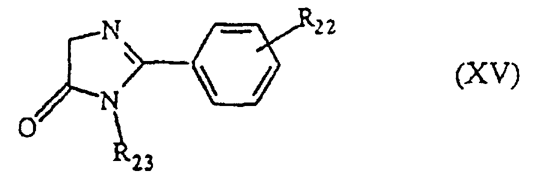 in which:
R₂₂ denotes a hydrogen atom or an alkyl group
R₂₃ denotes a hydrogen atom or an alkyl, alkoxy, nitro or nitrile group;
14) the dehydrobutyrolactone derivatives of formula (XVI) below: 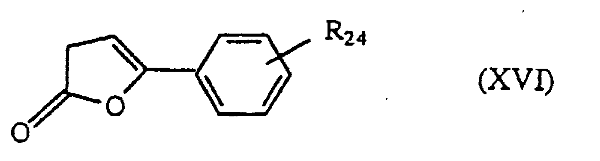 in which:
R₂₄ denotes a hydrogen atom or an alkyl, alkoxy, nitro or nitrile group;
15) the compounds of formula (XVII) below: 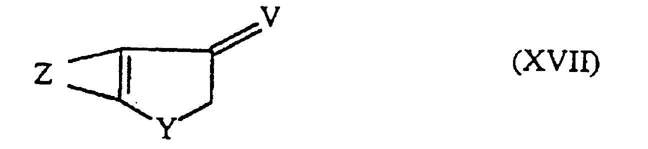 in which:
Z forms an aromatic ring
V denotes an oxygen atom or a group A-CH₂-E
in which A or E denotes a substituent having a Hammett constant of between 0.4 and 2.0 or substituents for which the sum of the Hammett constants is between 0.4 and 2.0
Y denotes Co, O, S or NR₁ when V is other than an oxygen atom, or denotes CS, C = NR₂, SO or SO₂ with R₁ or R₂ denoting a hydrogen atom or an alkyl radical;
and from the cosmetically acceptable salts of the compounds defined above;
and of at least one compound chosen from:
- one aldehyde corresponds to formula (XVIII) below: 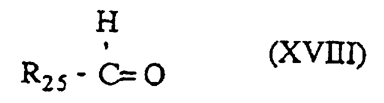 in which:
R₂₅ denotes a group of formula (XVIII A) below: 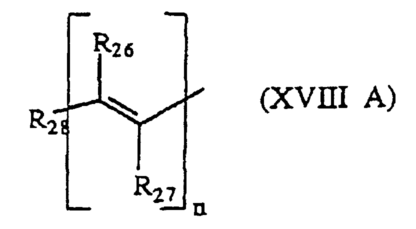
in which
R₂₆ and R₂₇, which are identical or different, denote a hydrogen atom or an alkyl, mono- or polyhydroxyalkyl, alkylhydroxyalkyl, alkoxy, -CF₃ or -OCF₃ group,
R₂₆ and R₂₇ can also form, together with the atoms to which they are attached, a 5- or 6-membered heterocyclic or aryl ring, it being possible for the said rings to be unsubstituted or substituted with a halogen, with a C₁-C₄ alkyl, a C₁-C₄ alkoxy, a nitro group, a hydroxyl group, a carboxyl group, a C₁-C₄ acyloxy group, a carboxamide group, a sulphonamide, sulpho, nitrile, -CF₃ or -OCF₃ group, or with a cycloalkyl or aryl radical which may be substituted with a C₁-C₄ alkyl;
n denotes an integer from 0 to 3,
R₂₈ denotes the substituents denoted by R₂₆, an aryl or alkylaryl group, the alkyl being unsubstituted or substituted with a halogen atom, a cyano or hydroxyl radical and the aryl being unsubstituted or substituted with a halogen, with a C₁-C₄ alkyl, a C₁-C₄ alkoxy, a nitro group, a hydroxyl group, a carboxyl group, a C₁-C₄ acyloxy group, a carboxamide group, a sulphonamide, sulpho, nitrile, -CF₃ or -OCF₃ group, or with a cycloalkyl or aryl radical which may be substituted with a C₁-C₄ alkyl;
a 5- or 6-membered heterocyclic group unsubstituted or substituted with a halogen, with a C₁-C₄ alkyl, a C₁-C₄ alkoxy, a nitro group, a hydroxyl group, a carboxyl group, a C₁-C₄ acyloxy group, a carboxamide group, a sulphonamide, sulpho, nitrile, -CF₃ or -OCF₃ group, or with a cycloalkyl or aryl radical which may be substituted with a C₁-C₄ alkyl;
or with the cosmetically acceptable salts of these compounds;
a ketone corresponds to formula (XIX) or (XX) below: 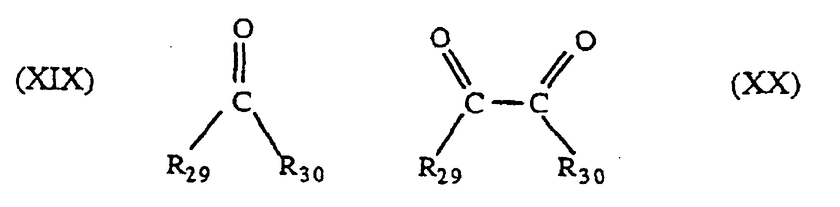 in which:
R₂₉ denotes the substituents denoted by R₂₅
R₃₀ denotes an alkyl, mono- or polyhydroxyalkyl, or alkylhydroxyalkyl group; an aryl or alkylaryl group, a 5- or 6-membered heterocycle unsubstituted or substituted with a halogen, with a C₁-C₄ alkyl, a C₁-C₄ alkoxy, a nitro group, a hydroxyl group, a carboxyl group, a C₁-C₄ acyloxy group, a carboxamide group, a sulphonamide, sulpho, nitrile, -CF₃ or -OCF₃ group, or with a cycloalkyl or aryl radical which may be substituted with a C₁-C₄ alkyl;
R₂₉ and R₃₀ can also form, together with the atoms to which they are attached, a 5- or 6-membered aryl ring, or a heterocycle comprising heteroatoms such as N or S, the said ring possibly being itself attached to a 5- or 6-membered aryl ring or to a heterocycle comprising heteroatoms such as N or S, it being possible for the said rings to be unsubstituted or substituted with a halogen, with a C₁-C₄ alkyl, a C₁-C₄ alkoxy, a nitro group, a hydroxyl group, a carboxyl group, a C₁-C₄ acyloxy group, a carboxamide group, a sulphonamide, sulpho, nitrile, -CF₃ or -OCF₃ group, or with a cycloalkyl or aryl radical which may be substituted with a C₁-C₄ alkyl; or with the cosmetically acceptable salts of these compounds,
- a quinone corresponding to formulae (XXI) and (XXII) below: 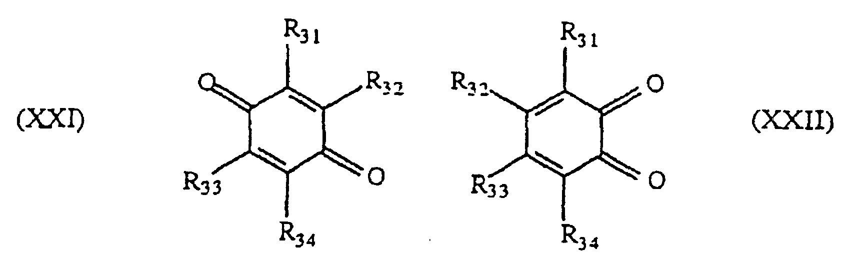 in which: R₃₁ denotes a hydrogen or halogen atom or a sulpho or alkoxy group, R₃₂, R₃₃ and R₃₄, which are identical or different, denote a hydrogen or halogen atom, a hydroxyl, alkyl, mono- or polyhydroxyalkyl, alkylhydroxyalkyl, alkylsulphonyl, carboxyalkyl, aminoalkyl, alkylaminoalkyl, (dihydroxy)alkylaminoalkyl or alkyl-NR'R" group, with R' and R" denoting alkyl or possibly forming, together with the nitrogen atom to which they are attached, an aryl ring or a 5- or 6-membered heterocycle, an aryl group, an amino group which may be substituted with an alkyl or a hydroxyalkyl; R₃₁ and R₃₂, R₃₁ and R₃₃ or R₃₃ and R₃₄ can form, together with the atoms to which they are attached, an aryl ring or a 5- or 6-membered heterocycle, unsubstituted or substituted with a halogen, with a C₁-C₄ alkyl, C₁-C₄ alkoxy, a nitro group, a hydroxyl group, a carboxyl group, a C₁-C₄ acyloxy group, a carboxamide group, a sulphonamide, sulpho, nitrile, -CF₃ or -OCF₃ group, or with a cycloalkyl or aryl radical which may be substituted with a C₁-C₄ alkyl;
or to the cosmetically acceptable salts of these compounds,
and a diiminoisoindoline or 3-aminoisoindolone derivative corresponding to the formula (XXIII) below: 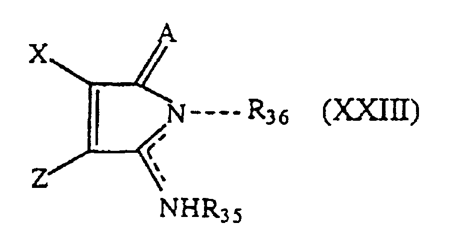 in which: R₃₅ and R₃₆, which are identical or different, denote a hydrogen atom, an alkyl, mono- or polyhydroxyalkyl, alkylhydroxyalkyl, aminoalkyl, alkylaminoalkyl, (dihydroxy)alkylaminoalkyl or an alkyl-NR'R" group, with R' and R" denoting alkyl or possibly forming, together with the nitrogen atom to which they are attached, an aryl ring or a 5- or 6-membered heterocycle, A denotes an oxygen atom or NH, X and Z together form an aryl ring or a 5- or 6-membered heterocycle, unsubstituted or substituted with a halogen, with a C₁-C₄ alkyl, a C₁-C₄ alkoxy, a nitro group, a hydroxyl group, a carboxyl group, a C₁-C₄ acyloxy group, a carboxamide group, a sulphonamide, sulpho, nitrile, -CF₃ or -OCF₃ group, or with a cycloalkyl or aryl radical which may be substituted with a C₁-C₄ alkyl;
or with the cosmetically acceptable salts of these compounds,
these compounds making it possible to obtain, by reaction without an oxidizing agent, a coloration of the said keratin fibres,
and also the combinations of the following compounds:
- 3-imino-3H-isoindol-1-ylamine and benzoylacetonitrile;
- 4-dimethylaminobenzaldehyde and benzoylacetonitrile;
- 1,4-naphthoquinone and benzoylacetonitrile;
- isatin and benzoylacetonitrile;
- 3-imino-3H-isoindol-1-ylamine and 5-amino-2H-pyrazol-3-ol;
- 4-dimethylaminobenzaldehyde and 5-amino-2H-pyrazol-3-ol;
- 1,4-naphthoquinone and 5-amino-2H-pyrazol-3-ol;
- 3-imino-3H-isoindol-1-ylamine and 3-amino-1-phenyl-2-pyrazolin-5-one;
- 4-dimethylaminobenzaldehyde and 3-amino-1-phenyl-2-pyrazolin-5-one;
- 1,4-naphthoquinone and 3-amino-1-phenyl-2-pyrazoline-5-one.

2. Composition for dyeing keratin fibres, in particular human keratin fibres such as the hair, **characterized in that** it comprises at least one compound containing active methylene chosen from the compounds defined according to Claim 1, and at least one compound chosen from an aldehyde, a ketone, a quinone and a diiminoisoindoline or 3-aminoisoindolone derivative according to Claim 1, in a medium which is suitable for dyeing.

3. Dye composition according to Claim 2, **characterized in that** it has a pH of between 2 and 11.

4. Composition according to Claim 2 or 3, **characterized in that** the compound containing active methylene is present in a concentration ranging from 0.01% to 10%, and preferably from 0.05% to 5%, by weight relative to the total weight of the composition.

5. Composition according to any one of Claims 2 to 4, **characterized in that** the compound chosen from an aldehyde, a ketone, a quinone and a diiminoisoindoline or 3-aminoisoindolone derivative is present in a concentration ranging from 0.01% to 10%, and preferably from 0.05% to 5%, by weight relative to the total weight of the composition.

6. Composition according to any one of Claims 2 to 5, **characterized in that** the medium which is suitable for dyeing is an aqueous medium consisting of water and/or organic solvents chosen from alcohols, glycols and glycol ethers, in proportions of between 0.5% and 20% by weight relative to the total weight of the composition.

7. Process for dyeing keratin fibres, in particular human keratin fibres such as the hair, **characterized in that** it consists in applying to the said fibres a component (A) consisting of a composition containing, in a medium which is suitable for dyeing, at least one compound containing active methylene as defined in Claim 1 and at least one component (B) consisting of a composition containing, in a medium which is suitable for dyeing, at least one compound chosen from an aldehyde, a ketone, a quinone and a diiminoisoindoline or 3-aminoisoindolone derivative, so as to allow the development of a coloration with the said keratin fibres.

8. Process according to Claim 7, **characterized in that** it consists in mixing components (A) and (B) just before use, in immediately applying the resulting composition to the keratin fibres and in leaving the composition to act on the fibres for 1 to 60 minutes and preferably for 1 to 30 minutes; the keratin fibres then being rinsed, washed with shampoo, rinsed again and then dried.

9. Process according to Claim 7 or 8, **characterized in that** it consists in applying component (A) to the keratin fibres, followed or preceded by application of component (B) to the said fibres, in leaving each component to act for 1 to 60 minutes and preferably for 1 to 30 minutes, and in optionally rinsing with water between each application; the keratin fibres then being rinsed, washed with shampoo, rinsed again and then dried.

10. Agent for dyeing keratin fibres, and in particular human keratin fibres such as the hair, **characterized in that** it separately comprises components (A) and (B) as defined in Claim 7; components (A) and (B) being intended to be either mixed immediately before use, or applied successively to the fibres to be treated.

11. Multi-compartment device or "dyeing kit", **characterized in that** it comprises at least two compartments, one of which contains component (A) as defined in Claim 7, and the second of which contains component (B) as defined in Claim 7.

12. Device according to Claim 11, **characterized in that** component (A) and/or component (B) is/are in the form of an anhydrous composition and **in that** it comprises a third compartment containing a cosmetically acceptable aqueous medium which is suitable for dyeing and which is intended to be mixed, before use, into one or both of the first compartments containing each component (A) or (B).
